(19) Europäisches Patentamt
European Patent Office
Office européen des brevets

(11) Veröffentlichungsnummer: **0 144 032**
**B1**

(12) # EUROPÄISCHE PATENTSCHRIFT

(45) Veröffentlichungstag der Patentschrift: **03.08.88**

(51) Int. Cl.⁴: **C 07 D 501/46**, C 07 D 501/18, A 61 K 31/545

(21) Anmeldenummer: **84113927.2**

(22) Anmeldetag: **17.11.84**

(54) **Cephalosporine und Verfahren zu ihrer Herstellung.**

(30) Priorität: **30.11.83 DE 3343208**

(43) Veröffentlichungstag der Anmeldung:
**12.06.85 Patentblatt 85/24**

(45) Bekanntmachung des Hinweises auf die Patenterteilung:
**03.08.88 Patentblatt 88/31**

(84) Benannte Vertragsstaaten:
**AT BE CH DE FR GB IT LI NL SE**

(56) Entgegenhaltungen:
**EP - A - 0 049 448**
**EP - A - 0 081 674**
**EP - A - 0 107 138**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Angerbauer, Rolf, Dr., Sterntalerweg 29, D-5600 Wuppertal 1 (DE)**
Erfinder: **Boberg, Michael, Dr., Untere Bergerheide 47, D-5600 Wuppertal 1 (DE)**
Erfinder: **Metzger, Karl Georg, Dr., Pahlkestrasse 75, D-5600 Wuppertal 1 (DE)**
Erfinder: **Zeiler, Hans-Joachim, Dr., Elsbeekerstrasse 46, D-5620 Velbert 15 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 144 032 B1

**Beschreibung**

Die Erfindung betrifft neue Cephalosporine, ihre Verwendung als Arzneimittel, insbesondere in der antibakteriellen Therapie sowie Verfahren zur ihrer Herstellung.

Cephalosporine, die als Acylseitenkette einen Aminothiazolyl-acrylsäurerest tragen, sind aus der EP-A 00 49 448 bekannt.

Durch die Erfindung werden Cephalosporine der allgemeinen Formel I und deren pharmazeutisch verträgliche Salze und deren unter physiologischen Bedingungen spaltbare Ester zur Verfügung gestellt,

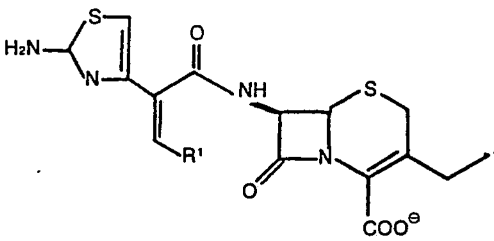

(I)

in der

$R^1$ $C_1$–$C_6$-Alkyl, Phenyl oder Halogen-substituiertes Phenyl bedeutet und

A einen Pyridiniumrest

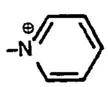

bedeutet, der 1- bis 3fach, gleich oder verschieden substituiert ist,

durch $C_1$–$C_6$-Alkyl, das ein- oder mehrfach substituiert ist durch Hydroxy, Carboxy, $C_1$–$C_6$-Alkyloxycarbonyl, Formyl oder $C_1$–$C_6$-Alkylcarbonyl, deren Carbonylgruppe auch in ketalisierter Form vorliegen kann, Carbamoyl, N-Hydroxy-carbamoyl, Sulfo, $C_1$–$C_6$-Alkyloxy, Hydroxy-$C_1$–$C_6$-alkyloxy, $C_1$–$C_6$-Alkylthio, $C_1$–$C_6$-Alkylsulfinyl, $C_1$–$C_6$-Alkylsulfonyl, $C_2$–$C_6$-Alkenyloxy, $C_2$–$C_6$-Alkenylthio, $C_2$–$C_6$-Alkenylsulfinyl oder $C_2$–$C_6$-Alkenylsulfonyl,

durch Cyano-$C_1$–$C_3$-alkyl, Epoxy-$C_2$–$C_6$-alkyl, Trifluormethyl oder Pentafluorethyl,

durch Hydroximino-methyl oder $C_1$–$C_4$-Alkoximinomethyl,

durch $C_2$–$C_6$-Alkenyl, das durch Hydroxy substituiert sein kann,

durch $C_2$–$C_6$-Alkinyl,

durch $C_3$–$C_7$-Cycloalkyl oder $C_3$–$C_7$-Cycloalkylmethyl, wobei in beiden Substituenten der Ring auch substituiert sein kann durch Hydroxy, Halogen, Carboxy, $C_1$–$C_6$-Alkyloxycarbonyl oder Cyano,

durch $C_4$–$C_7$-Cycloalkenyl,

durch $C_1$–$C_6$-Alkoxy, das durch Hydroxy, Carboxy oder $C_1$–$C_6$-Alkoxycarbonyl substituiert sein kann,

durch Epoxy-$C_2$–$C_6$-Alkoxy,

durch $C_2$–$C_6$-Alkenyloxy oder $C_2$–$C_6$-Alkinyloxy,

durch Phenoxy,

durch Amino, das gegebenenfalls ein- oder zweimal substituiert sein kann, durch $C_1$–$C_6$-Alkyl, Formyl, $C_1$–$C_6$-Alkylcarbonyl, $C_1$–$C_6$-Alkoxycarbonyl, Carbamoyl, $C_1$–$C_6$-Alkylsulfonyl,

durch Cyano, Hydroxy oder Mercapto,

durch $C_1$–$C_6$-Alkylthio, $C_1$–$C_6$-Alkylsulfinyl oder $C_1$–$C_6$-Alkylsulfonyl, die im Alkylteil durch Hydroxy substituiert sein können,

durch Methylthio, Methylsulfinyl oder Methylsulfonyl, die im Methylteil substituiert sind durch Carboxy oder $C_1$–$C_6$-Alkyloxycarbonyl,

durch $C_2$–$C_6$-Alkenylthio, $C_2$–$C_6$-Alkenylsulfinyl oder $C_2$–$C_6$-Alkenylsulfonyl,

durch Phenyl, Benzyl, die auch durch Halogen substituiert sein können,

durch Sulfamoyl, das am Stickstoff einmal substituiert sein kann, durch $C_1$–$C_6$-Alkylaminocarbonyl, an den ein oder zwei gegebenenfalls substituierte 3- bis 7gliedrige Ringe ankondensiert sein können, die jeweils bis zu zwei Heteroatome aus der Gruppe O, N und S und bis zu zwei Doppelbindungen enthalten können und auch aromatisch oder heteroaromatisch sein können, wobei folgende Substituenten in Betracht kommen:

$C_1$–$C_6$-Alkyl, $C_3$–$C_7$-Cycloalkyl, $C_1$–$C_4$-Alkoxy, $C_1$–$C_4$-Hydroxyalkyl, Halogen, Hydroxy, Oxo, Hydroximino, Exomethylen, Carboxy, $C_1$–$C_6$-Alkyloxycarbonyl, Cyano, Carbamoyl, Sulfamoyl, Amino, $C_1$–$C_4$-Alkylamino oder $C_1$–$C_4$-Dialkylamino.

Die Erfindung ist insbesondere auf Verbindungen gerichtet, in denen $R^1$ die vorstehende Bedeutung besitzt und

A eine Pyridiniumrest

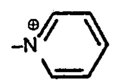

bedeutet, der 1- bis 3fach, gleich oder verschieden substituiert ist,

durch $C_1$–$C_6$-Alkyl, das ein- oder mehrfach substituiert ist, durch Hydroxy, Carboxy, $C_1$–$C_6$-Alkyloxycarbonyl, Formyl oder $C_1$–$C_6$-Alkylcarbonyl, deren Carbonylgruppe auch in ketalisierter Form vorliegen kann, Carbamoyl, N-Hydroxy-carbamoyl, Sulfo, $C_1$–$C_6$-Alkyloxy, Hydroxy-$C_1$–$C_6$-alkyloxy, $C_1$–$C_6$-Alkylthio, $C_1$–$C_6$-Alkylsulfinyl, $C_1$–$C_6$-Alkylsulfonyl, $C_2$–$C_6$-Alkenyloxy, $C_2$–$C_6$-Alkenylthio, $C_2$–$C_6$-Alkenylsulfinyl oder $C_2$–$C_6$-Alkenylsulfonyl,

durch Cyano-$C_1$–$C_3$-alkyl, Epoxy-$C_2$–$C_6$-alkyl, Hydroxyiminomethyl, $C_1$–$C_4$-Alkoxyiminomethyl, Trifluormethyl oder Pentafluorethyl, durch $C_2$–$C_6$-Alkenyl, das durch Hydroxy substituiert sein kann, durch $C_2$–$C_6$-Alkinyl, durch $C_3$–$C_7$-Cycloalkyl oder $C_3$–$C_7$-Cycloalkylmethyl, wobei in beiden Substituenten der Ring auch substituiert sein kann durch Hydroxy, Halogen, Carboxy, $C_1$–$C_6$-Alkyloxycarbonyl oder Cyano,

durch $C_4$–$C_7$-Cycloalkenyl

durch $C_1$–$C_6$-Alkoxy, das durch Hydroxy, Carboxy oder $C_1$–$C_6$-Alkyloxycarbonyl substituiert sein kann,

durch Expoxy-$C_2$–$C_6$-alkoxy,

durch $C_2$–$C_6$-Alkenyloxy oder $C_2$–$C_6$-Alkinyloxy,

durch Phenoxy,

durch Amino, das ein- oder zweifach, gleich oder verschieden substituiert sein kann durch $C_1$–$C_6$-Alkyl, Formyl, $C_1$–$C_6$-Alkylcarbonyl, $C_1$–$C_6$-Alkoxycarbonyl, Carbamoyl, $C_1$–$C_6$-Alkylsulfonyl,

durch Cyano, Hydroxy oder Mercapto,

durch $C_1$–$C_6$-Alkylthio, $C_1$–$C_6$-Alkylsulfinyl oder $C_1$–$C_6$-Alkylsulfonyl, die im Alkylteil durch Hydroxy substituiert sein können,

durch Methylthio, Methylsulfinyl oder Methylsulfonyl, die im Methylteil substituiert sind durch Carboxy oder $C_1$–$C_6$-Alkyloxycarbonyl,
durch $C_2$–$C_6$-Alkenylthio, $C_2$–$C_6$-Alkenylsulfinyl oder $C_2$–$C_6$-Alkenylsulfonyl,
durch Phenyl, Benzyl, die auch durch Halogen substituiert sein können,
durch Formyl oder ketalisiertes Formyl,
durch $C_1$–$C_6$-Alkylcarbonyl, das auch durch Hydroxy substituiert sein und in ketalisierter Form vorliegen kann,
durch $C_1$–$C_6$-Alkylcarbonylamino,
durch Carboxy oder $C_1$–$C_6$-Alkoxycarbonyl,
durch Sulfamyl, das am Stickstoff einmal substituiert sein kann durch $C_1$–$C_6$-Alkylaminocarbonyl, an den ein gegebenenfalls substituierter 3- bis 7gliedriger, bevorzugt 5- oder 6gliedriger Ring ankondensiert sein kann, der bis zu zwei Heteroatome aus der Gruppe O, N, S und bis zu zwei Doppelbindungen enthalten kann und auch aromatisch oder heteroaromatisch sein kann und der durch folgende Substituenten ein- oder mehrfach, vorzugsweise jedoch einfach, substituiert sein kann,
$C_1$–$C_6$-Alkyl, $C_3$–$C_7$-Cycloalkyl, $C_1$–$C_4$-Alkoxy, $C_1$–$C_4$-Hydroxyalkyl, Halogen, Hydroxy, Oxo, Hydroximino, Exomethylen, Carboxy, $C_1$–$C_6$-Alkyloxycarbonyl, Cyano, Carbamoyl, Sulfamoyl, Amino, $C_1$–$C_4$-Alkylamino oder $C_1$–$C_4$-Dialkylamino.

Als ganz besonders bevorzugt kommen Verbindungen der allgemeinen Formel I in Betracht, in der $R^1$ für $C_1$–$C_5$-Alkyl, insbesondere Methyl steht und A ein Pyridiniumrest ist, der 1- bis 2fach substituiert ist, durch Hydroxy-$C_1$–$C_4$-alkyl, wie insbesondere Hydroxymethyl, Hydroxyethyl, Hydroxypropyl, Hydroxyisopropyl, Hydroxybutyl, Hydroxy-sec.-butyl oder Hydroxy-tert.-butyl, und wobei z.B. auch zwei oder drei Hydroxylgruppen am Alkylrest stehen können, Carboxy-$C_1$–$C_4$-alkyl, wie insbesondere Carboxymethyl und Carboxyethyl,
$C_1$–$C_4$-Alkyloxycarbonyl-$C_1$–$C_4$-alkyl, wie insbesondere Methyloxycarbonylmethyl, Ethyloxycarbonylmethyl, Methyloxycarbonylethyl, Formyl-$C_1$–$C_4$-alkyl, wie insbesondere Formylmethyl, $C_1$–$C_4$-Alkylcarbonyl-$C_1$–$C_4$-alkyl, wie insbesondere
Methylcarbonylmethyl, Ethylcarbonylmethyl, Methylcarbonylethyl und Ethylcarbonylethyl, deren beide Alkylgruppen auch noch durch Hydroxy substituiert sein können und deren Carbonylgruppe auch in ketalisierter Form vorliegen kann,
durch Carbamoyl substituiertes $C_1$–$C_4$-Alkyl, wie insbesondere Carbamoylmethyl und Carbamoylethyl, die am Stickstoff auch noch durch Hydroxy substituiert sein können, wie insbesondere N-Hydroxycarbamoylmethyl, Sulfo-$C_1$–$C_4$-alkyl, wie insbesondere Sulfoethyl oder 1-Hydroxy-1-sulfomethyl,
$C_1$–$C_4$-Alkyloxy-$C_1$–$C_4$-alkyl, wie insbesondere Methyloxymethyl, Ethyloxymethyl, Propyloxymethyl, Isopropyloxymethyl, Methyloxyethyl, Ethyloxyethyl, Methyloxypropyl und Methyloxyisopropyl, die auch durch Hydroxy substituiert sein können, wie insbesondere Hydroxyethyloxymethyl und Hydroxyethyloxyethyl,
$C_1$–$C_4$-Alkylthio-$C_1$–$C_4$-alkyl, wie insbesondere Methylthiomethyl, Ethylthiomethyl, Methylthioethyl und Ethylthioethyl,
$C_1$–$C_4$-Alkylsulfinyl-$C_1$–$C_4$-alkyl, wie insbesondere Methylsulfinylmethyl, Ethylsulfinylmethyl, Methylsulfinylethyl und Ethylsulfinylethyl,
$C_1$–$C_4$-Alkylsulfonyl-$C_1$–$C_4$-alkyl, wie insbesondere Methylsulfonylmethyl, Ethylsulfonylmethyl, Methylsulfonylethyl und Ethylsulfonylethyl,
$C_3$-Alkenyloxy-$C_1$–$C_4$-alkyl, wie insbesondere Allyloxymethyl und Allyloxyethyl,
$C_3$-Alkenylthio-$C_1$–$C_4$-alkyl, wie insbesondere Allylthiomethyl,
$C_3$-Alkenylsulfinyl-$C_1$–$C_4$-alkyl, wie insbesondere Allylsulfinylmethyl,
$C_3$-Alkenylsulfonyl-$C_1$–$C_4$-alkyl, wie insbesondere Allylsulfonylmethyl,
Cyano-$C_1$–$C_3$-alkyl, wie insbesondere Cyanomethyl und Cyanoethyl,
Epoxy-$C_2$–$C_3$-alkyl, wie insbesondere Epoxyethyl und Epoxypropyl,
Trifluormethyl, Hydroximinomethyl und $C_1$–$C_3$-Alkyloximinomethyl, wie insbesondere Methoximinomethyl,
$C_3$–$C_4$-Alkenyl, wie insbesondere Allyl, 2-Methylallyl und Buten-3-yl, die auch noch durch Hydroxy substituiert sein können, wie insbesondere Hydroxyallyl und Hydroxybutenyl,
$C_3$-Alkinyl, wie insbesondere Propargyl,
$C_3$–$C_6$-Cycloalkyl und $C_3$–$C_6$-Cycloalkyl-methyl, wie insbesondere Cyclopropyl, Cyclobutyl, Cyclopentyl, Cyclohexyl und Cyclopentylmethyl, wobei die Ringe auch substituiert sein können, z.B. durch Hydroxy, wie insbesondere 1-Hydroxy-1-cyclopentyl und 1-Hydroxy-1-cyclohexyl, oder durch Halogen, vorzugsweise Chlor, durch Carboxy, $C_1$–$C_4$-Alkoxycarbonyl oder Cyano,
$C_5$–$C_6$-Cycloalkenyl, wie insbesondere Cyclopenten-1-yl und Cyclohexen-1-yl,
$C_1$–$C_4$-Alkoxy, wie insbesondere Methoxy, Ethoxy, Propoxy, Isopropoxy, Butoxy, Isobutoxy und tert.-Butoxy, vorzugsweise Methoxy, wobei diese Alkoxygruppen auch noch substituiert sein können, beispielsweise durch Hydroxy, Carboxy oder $C_1$–$C_4$-Alkyloxycarbonyl, insbesondere Carboxymethyloxy und Methyloxycarbonylmethyloxy,
Epoxy-$C_2$–$C_3$-alkyloxy, wie insbesondere Epoxyethoxy oder Epoxypropoxy,
$C_3$-Alkenyloxy, wie insbesondere Allyloxy,
$C_3$-Alkinyloxy, wie insbesondere Propargyloxy, Phenoxy, Amino,
$C_1$–$C_5$-Alkylamino, wie insbesondere Ethylamino,
$C_1$–$C_5$-Dialkylamino, wie insbesondere Dimethylamino und Diethylamino,
$C_1$–$C_4$-Alkoxycarbonylamino, wie insbesondere Methoxycarbonylamino und Ethoxycarbonylamino,
$C_1$–$C_4$-Alkylcarbonylamino, wie insbesondere Methylcarbonylamino,
N-$C_1$–$C_4$-Alkyl- und Dialkylcarbamoylamino, wie insbesondere N-Methylcarbamoylamino, N,N-Diethylcarbamoylamino,
$C_1$–$C_4$-Alkylsulfonylamino, wie insbesondere Methyl- oder Ethylsulfonylamino, Cyano, Hydroxy, insbesondere 3-Hydroxy,
$C_1$–$C_4$-Alkylthio, wie insbesondere Methylthio, Ethylthio, Propylthio und Isopropylthio, die auch substituiert sein können durch Hydroxy, insbesondere Hydroxyethylthio,

$C_1$–$C_4$-Alkylsulfinyl, wie insbesondere Methylsulfinyl, Ethylsulfinyl, Propylsulfinyl und Isopropylsulfinyl, die auch substituiert sein können durch Hydroxy, insbesondere Hydroxyethylsulfinyl,

$C_1$–$C_4$-Alkylsulfonyl, wie Methyl- oder Ethyl- oder Propyl- oder Isopropylsulfonyl, die auch substituiert sein können durch Hydroxy, insbesondere Hydroxyethylsulfonyl,

Carboxymethylthio und $C_1$–$C_4$-Alkyloxycarbonylmethylthio, insbesondere Methyloxycarbonylmethylthio,

Carboxymethyl-sulfinyl und -sulfonyl, sowie $C_1$–$C_4$-Alkyloxycarbonylmethyl-sulfinyl und -sulfonyl, insbesondere Methyloxycarbonylmethyl-sulfinyl und -sulfonyl,

$C_3$-Alkenylthio, wie Allylthio und Propen-1-ylthio,

$C_3$-Alkenylsulfinyl, wie Allylsulfinyl und Propen-1-sulfinyl,

$C_3$-Alkenylsulfonyl, wie Allylsulfonyl und Propen-1-ylsulfonyl,

Phenyl und Benzyl, die auch durch Halogen, insbesondere Chlor, substituiert sein können, wie z.B. 4-Chlorbenzyl,

2′-Thienyl und 3′-Thienyl,

Formyl und ketalisiertes Formyl, wie z.B. 1,3-Dioxolan-2-yl,

$C_1$–$C_4$-Alkylcarbonyl, insbesondere Acetyl und Propionyl, vorzugsweise Acetyl, die auch die Hydroxy substituiert sein und in ketalisierter Form vorliegen können, wie z.B. 2-Methyl-1,3-dioxolan-2-yl, Benzoyl,

$C_1$–$C_4$-Alkylcarbonylamino, insbesondere Acetylamino und Propionylamino, Formylamino, Carboxy, $C_1$–$C_4$-Alkoxycarbonyl, insbesondere Methoxycarbonyl und Ethoxycarbonyl,

und an den ein gegebenenfalls, wie oben erwähnt, substituierter 5- oder 6gliedriger Ring ankondensiert sein kann, der bis zu zwei Heteroatome aus der Gruppe O, N und S und bis zu zwei Doppelbindungen enthalten kann und der auch aromatisch oder heteroaromatisch sein kann. Als ankondensierte Ringe kommen insbesondere folgende Ringsysteme in Betracht:

Cyclopenteno, Dihydrocyclopenteno, Cyclohexeno, Dehydrocyclohexeno,

Benzo,

Furo, Dihydrofuro,

Pyrano, Dihydropyrano,

Thieno, Dihydrothieno,

Thiopyrano, Dihydrothiopyrano,

Pyrido, Dihydropyrido, Tetrahydropyrido,

Pyrimido, Dihydropyrimido, Tetrahydropyrimido,

Pyrazino, Dihydropyrazino, Tetrahydropyrazino,

Pyridazino, Dihydropyridazino, Tetrahydropyridazino, die jeweils ein- oder mehrfach, vorzugsweise jedoch einfach, substituiert sein können, vorzugsweise durch

$C_1$–$C_4$-Alkyl, wie insbesondere Methyl, Ethyl und Isopropyl,

$C_3$–$C_6$-Cycloalkyl, wie insbesondere Cyclopropyl,

$C_1$–$C_4$-Alkoxy, wie insbesondere Methoxy und Ethoxy,

$C_1$–$C_3$-Hydroxyalkyl, wie insbesondere Hydroxymethyl, Hydroxyethyl,

Halogen, wie insbesondere Chlor und Fluor, Hydroxy, Carboxy und Cyano,

$C_1$–$C_6$-Alkyloxycarbonyl, wie insbesondere Methoxycarbonyl und Ethoxycarbonyl, Oxo und Hydroximino, Carbamoyl und Sulfamoyl,

Amino, $C_1$–$C_4$-Alkylamino, wie insbesondere Methylamino und Ethylamino, $C_1$–$C_4$-Dialkylamino, wie insbesondere Diethylamino.

Die Verbindungen der allgemeinen Formel I erhält man, indem man die Säuren der Formel II, worin $R^2$ eine übliche Schutzgruppe darstellt, in die gemischten Anhydride der Formel III überführt, diese mit den Verbindungen der Formel IV umsetzt und anschliessend von den so erhaltenen Verbindungen der Formel V die Schutzgruppe $R^2$ abspaltet.

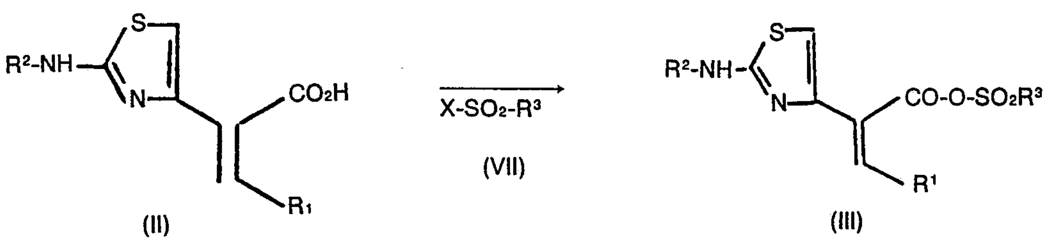


(II) (III)

III +
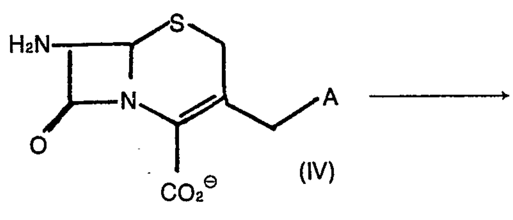

(IV)

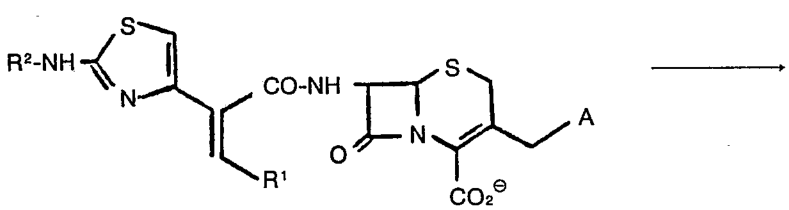

I

(V)

Für das Verfahren ist es vorteilhaft, als Schutzgruppe $R^2$ eine säurelabile Schutzgruppe wie z.B. tert.-Butyloxycarbonyl, Trityl oder Formyl zu verwenden und die Abspaltung von $R^2$ in V zur Herstellung der erfindungsgemässen Verbindungen I mit einer Säure, z.B. Trifluoressigsäure oder Ameisensäure vorzunehmen.

Die Verbindungen II und III lassen sich nach folgendem Formelschema herstellen.

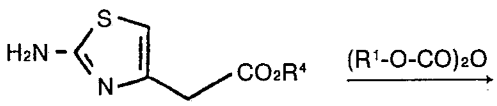


(IX)

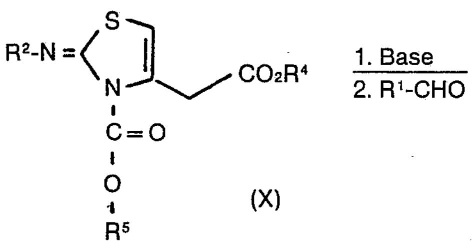


(X)

(Bei diesem Verfahren zur Herstellung von II ist $R^2 = R^5\text{–O–CO}$)

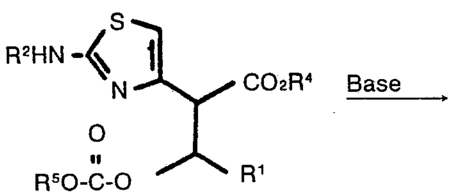


(XI)

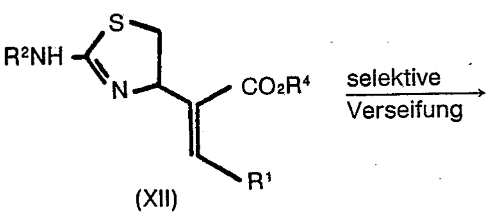


(XII)

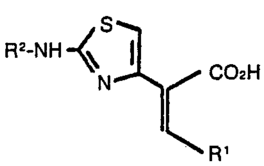

\+

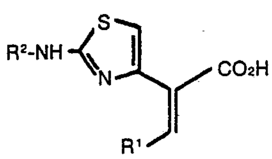

(II) (IIa)

[ IIa $\xrightarrow[\text{2. Base}]{\text{1. Silylierung}}$ II + IIa $\xrightarrow{\text{Trennung}}$ II ]

II 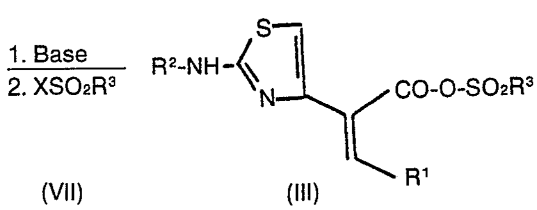


(VII) (III)

Zunächst werden die Verbindungen der Formel IX [siehe z.B. E. Campaigne und T.P. Selby, J. Heterocycl. Chem. 17 (1980), 1255] zu den Verbindungen der Formel X umgesetzt.

In Formel X bedeuten

$R^2$ eine Aminschutzgruppe wie z.B. Acetyl, Benzoyl, Formyl, Trichloracetyl, Benzyloxycarbonyl, Methyloxycarbonyl oder tert.-Butyloxycarbonyl und

$R^4$ und $R^5$, die verschieden oder gleich sein können, einen gegebenenfalls substituierten Alkyl-, Cycloalkyl-, Alkenyl-, Cycloalkenyl-, Aryl- oder Heterocyclylrest, wobei Heteroatome in den Heterocyclylresten sowie Doppelbindungen in den Alkenyl- und Cycloalkenylresten mindestens durch ein C-Atom von der Oxycarbonylgruppe getrennt sind.

Besonders sind

$R^4$ und $R^5$ ein gegebenenfalls substituierter Alkylrest mit 1 bis 15 C-Atomen, ein gegebenenfalls substituierter Alkenylrest mit 3 bis 15 C-Atomen, ein gegebenenfalls substituierter Cycloalkylrest mit 3 bis 10 C-Atomen, ein gegebenenfalls substituierter Cycloalkenylrest mit 5 bis 10 C-Atomen, ein gegebenenfalls substituierter Arylrest mit 1 bis 3 Ringen oder ein gegebenenfalls substituierter Heterocyclylrest mit 1 bis 3 Ringen, die bis zu 5 Stickstoff-, Schwefel- oder Sauerstoffatome enthalten können.

Die genannten Alkyl-, Alkenyl-, Cycloalkyl- und Cycloalkenylreste $R^4$ und $R^5$ können durch Alkylreste mit 1 bis 4 C-Atomen, O-Alkylreste mit 1 bis C-Atomen, Halogen, vorzugsweise Chlor, gegebenenfalls substituierte Phenylreste, C≡N und $C_1$–$C_5$-Trialkylsilyl substituiert sein.

Alle Aryl- und Heterocyclylreste $R^4$ und $R^5$ einschließlich der genannten Phenylreste können durch Alkyl-, O-Alkyl-, S-Alkyl-, Alkyloxycarbonyl-, Halogen- und Phenylreste substituiert sein, wobei alle Alkylreste 1 bis 4 C-Atome haben können, sowie durch Nitro und C≡N.

Wenn die Reste $R^4$ und/oder $R^5$ substituiert sind, vorzugsweise durch die obengenannten Substituenten, so können sie 1 bis 5, vorzugsweise 1 oder 2 Substituenten tragen.

Besonders vorteilhaft für das Verfahren ist es, wenn

$R^2$ = eine säurelabile Schutzgruppe wie z.B. tert.-Butoxycarbonyl ist, und wenn

$R^4$ = ein basisch verseifbarer Rest wie z.B. Methyl oder Ethyl ist.

Die Verbindungen der Formel X erhält man, indem man die an sich bekannten Verbindungen der Formel IX in einem geeigneten Lösungsmittel mit einem Pyrokohlensäureester der Formel $R^5$–O–CO–O–CO–O–$R^5$ reagieren läßt.

Als Lösungsmittel für diese Reaktion eignen sich besonders aprotische, polare Lösungsmittel wie z.B. Acetonitril, Dimethylformamid, Hexamethylphosphorsäuretriamid oder Dimethylsulfoxid, besonders die beiden letzten. Die Reaktion läuft besonders vorteilhaft bei Zimmertemperatur oder bei niedrigeren Temperaturen z.B. 0 bis –50 °C ab, wobei man die Komponenten 1 bis 7 Tage miteinander reagieren läßt. Der Pyrokohlensäureester wird im algemeinen mit 2 bis 2,5 Moläquivalenten eingesetzt.

Zur Herstellung der Verbindungen der Formel XI versetzt man die Verbindungen der Formel X in einem geeigneten Lösungsmittel bei tiefen Temperaturen mit 1 bis 1,1 Äquivalenten einer Base und gibt anschliessend 1 bis 1,1 Äquivalente eines Aldehyds der Formel $R^1$–CHO hinzu.

Als Lösungsmittel für die Reaktion können z.B. Dimethylformamid, Dimethylsulfoxid, Diethylether, Tetrahydrofuran oder Toluol – bevorzugt Tetrahydrofuran – und als Base Alkoholate, Hydride, Amide oder Metallorganyle – bevorzugt Kalium-tert.-Butylat, Lithiumdiisopropylamid und Butyllithium – verwendet werden. Zur Durchführung der Reaktion gibt man die Base bei –50 bis –80 °C zu einer Lösung von X und fügt anschließend bei –50 bis –60 °C den Aldehyd hinzu und rührt etwa 12 Stunden bei –50 bis –60 °C. Zur Isolierung der Produkte der Formel XI wird der Ansatz neutralisiert und aufgearbeitet.

In den Verbindungen der Formel XI haben $R^2$, $R^4$ und $R^5$ die bei den Verbindungen der Formel X aufgeführten Bedeutungen, und $R^1$ hat die eingangs genannte Bedeutung.

Zur Durchführung des Verfahrens zur Herstellung der Verbindungen der allgemeinen Formel I ist es nicht notwendig, die Verbindungen der Formel XI zu isolieren. Es ist vielmehr zweckmäßig, sie in situ direkt in die Verbindungen der Formel XII umzuwandeln. Hierzu reicht es im allgemeinen aus, den Ansatz nach Zugabe des Aldehyds $R^1$–CHO auf Raumtemperatur erwärmen zu lassen und über Nacht bei Raumtemperatur zu rühren. Sollte bis dahin die Eliminierung von XI nach XII nicht vollständig sein, gibt man 1 bis 1,2 Äquivalente einer Base – wie z.B. ein Hydrid, ein Alkoholat oder ein Amid – besonders Kalium-tert.-butylat hinzu und rührt etwa 10 h bei Raumtemperatur.

Hatte man dagegen die Verbindung der Formel XI zuvor isoliert, so gibt man zur Herstellung der Verbindungen der Formel XII zu einer Lösung der Verbindungen der Formel XI in einem geeigneten Lösungsmittel, 1,1 bis 2,2 Äquivalente einer Base. Als Lösungsmittel und als Base können die bei der Umsetzung von X nach XI genannten Verwendung finden, bevorzugt Tetrahydrofuran und Kalium-tert.-butylat.

Man erhält die Verbindungen der Formel XII als E/Z-Isomerengemische, die sich z.B. durch Umkristallisieren oder durch Säulenchromatographie an Kieselgel trennen lassen.

In den Verbindungen der Formel XII haben $R^1$, $R^2$ und $R^4$ die gleiche Bedeutung wie in den Verbindungen der Formel XI.

Zur Herstellung der Z-Carbonsäuren der Formel II kann man die Z-Ester, die man durch Trennung der E/Z-Isomerengemische der Ester der Formel XII erhalten kann, verseifen. Es ist aber für die Durchführung des Verfahrens zur Herstellung der Verbindungen der allgemeinen Formel I günstiger, das E/Z-Isomerengemisch der Ester der Formel XII in der Weise selektiv zu verseifen, daß man unter milden Bedingungen erst die E-Ester in die E-Carbonsäuren der Formel IIa überführt und abtrennt und anschließend die verbleibenden Z-Ester, in denen die Estergruppe sterisch stärker abgeschirmt ist, unter drastischen Bedingungen zu den Z-Carbonsäuren der Formel II verseift.

Die milden Verseifungsbedingungen, die zu den E-Carbonsäuren IIa führen, sind z.B. Ethanol/2 N Natronlauge/Raumtemperatur/24 h. Zweckmäßigerweise führt man die Verseifung in der Art durch, daß man nach der Überführung der Verbindungen der Formel XI in die Verbindungen der XII direkt zu dem Reaktionsansatz 2 N Natronlauge gibt und bei Raumtemperatur oder unter leichtem Erwärmen so lange rührt, bis die E-Ester verseift sind. Dann trennt man durch Extraktion im Alkalischen die Z-Ester von dem Ansatz ab und verseift sie unter drastischeren Bedingungen.

Drastischere Verseifungsbedingungen sind z.B. Ethanol/2 N Natronlauge/24 h Rückfluß – eventuell auch stärkere Natronlauge oder höherer siedende Lösungsmittel wie z.B. Dioxan.

Auf diese Weise erhält man die gewünschte Z-Carbonsäuren der Formel II und die E-Carbonsäuren der Formel IIa. Letztere lassen sich nach Überführung in die Silylester – z.B. mit Bistrimethylsilylacetamid – in einem geeigneten Lösungsmittel – z.B. Diethylether oder Terahydrofuran – mit einer Base wie Kalium-tert.-butylat und anschließender Hydrolyse mit verdünnter Säure wieder in ein Gemisch der E-Carbonsäuren der Formel IIa und der Z-Carbonsäuren der Formel II überführen.

Aus diesem E/Z-Isomerengemisch lassen sich die Z-Carbonsäuren der Formel II z.B. durch Kristallisation oder durch Trennung an einem Ionenaustauscher rein isolieren.

Die Trennung mit Hilfe von Ionenaustauschern ist einfach, da die Z-Carbonsäuren der Formel II sehr viel stärker sauer sind als die E-Carbonsäuren der Formel IIa. So werden die E-Carbonsäuren der Formel IIa bereits mit Methanol von schwach basischen Ionenaustauschern eluiert, die Z-Carbonsäuren der Formel II dagegen erst nach Zusatz von Elektrolyten, z.B. 2 N Natronlauge. Unter schwach basischen Ionenaustauschern sind solche Ionenaustauscher in fester oder flüssiger Form zu verstehen, die tertiäre Aminogruppen enthalten wie z.B. Lewatit® MP 62.

In den Verbindungen der Formel II und IIa haben $R^1$ und $R^4$ die gleiche Bedeutung wie bei den Verbindungen der Formel XII. Zusätzlich kann $R^4 = H$ sein, wenn vor der Verseifung in den Verbindungen der Formel XII $R^4$ eine alkalisch verseifbare Schutzgruppe wie z.B. Acetyl war. Für die Durchführung des Verfahrens zur Herstellung der Verbindungen der allgemeinen Formel I ist es aber zweckmäßiger, wenn $R^4$ eine Schutzgruppe ist, die unter den Verseifungsbedingungen stabil ist – bevorzugt tert.-Butyloxycarbonyl.

Die Verbindungen der Formel IV erhält man, indem man Cephalosporine der Formel XIII, worin $R^6$ eine übliche Schutzgruppe darstellt, mit ungeladenen stickstoffhaltigen Heterocyclen A′ umsetzt, wobei A′ die obengenannte Bedeutung von A hat, jedoch die positive Ladung am Stickstoff nicht trägt und anschließend in den erhaltenen Verbindungen der Formel XIV die Schutzgruppe $R^6$ abspaltet.

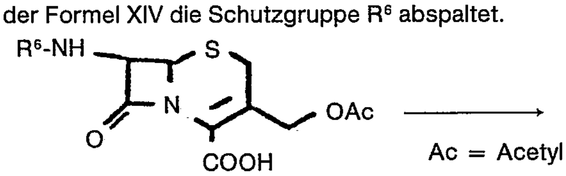

Ac = Acetyl

(XIII)

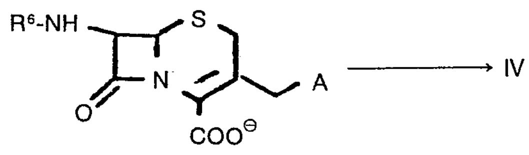


(XIV)

Für das Verfahren ist es vorteilhaft, wenn $R^6$ eine Schutzgruppe darstellt, die sich entweder nach der in der β-Lactamchemie üblichen Phosphorsäurechloridmethode oder enzymatisch abspalten läßt.

Die Umsetzung von Verbindungen der Formel XIII zu Verbindungen der Formel XIV erfolgt in organischen oder wäßrigen Lösungsmitteln, bei Temperaturen zwischen 20 und 100 °C, vorzugsweise zwischen 60 und 80 °C, unter Zugabe eines geeigneten Salzkatalysators. Dabei wird als Reagenz eine ungeladene Verbindung der Formel A' eingesetzt, in der A' die eingangs genannte Bedeutung hat.

Geeignete Salzkatalysatoren sind zum Beispiel NaBr, KJ, KSCN, NaSCN, LiJ. Ein bevorzugtes organisches Lösungsmittel ist Dimethylformamid. Besonders bevorzugt ist die Reaktion in wäßriger Lösung unter Verwendung eines großen Überschusses KSCN. Es ist günstig, die Verbindung A' in geringem Überschuß einzusetzen, wobei der Zusatz von 1 Äquivalent anorganischer Base, bevorzugt Natriumhydrogencarbonat, vorteilhaft ist.

Die Abspaltung der Schutzgruppe $R^6$ in Formel XIII erfolgt entweder nach literaturbekannter Weise mit Phosphorpentachlorid oder bevorzugt enzymatisch mit Penicillin-Acylase. Für die enzymatische Abspaltung ist es besonders vorteilhaft, wenn $R^6$ Phenylacetyl oder Thienylacetyl ist. Die Abspaltung erfolgt vorteilhaft in wäßriger Lösung bei pH 7 bis 8.

Für die Kupplung von Carbonsäuren an 7-Aminocephalosporansäuren sind in der Cephalosporinchemie eine große Anzahl von Methoden bekannt, die sich letztlich aus der Peptidchemie ableiten. Bei den Versuchen zur Knüpfung der Amidbindung zwischen den Z-Carbonsäuren der Formel II und den Cephalosporansäuren der Formel IV versagen diese Methoden jedoch oder führen nur zu sehr schlechten Ausbeuten, insbesondere dann, wenn $R^1$ ein Alkylrest ist. Die Gründe hierfür sind in der grossen sterischen Hinderung der Carboxylgruppe in den Carbonsäuren der Formel II durch den Rest $R^1$ sowie in der ausgeprägten Neigung des Restes $R^1$, nach Aktivierung der Carboxylfunktion – z.B. Überführung in das Säurechlorid – in die E-Form zu isomerisieren, zu suchen.

Man kann aber die Z-Carbonsäuren der Formel II auf einfache, schonende und billige Weise aktivieren, wenn man sie bei tiefen Temperaturen in die gemischten Anhydride der Formel III überführt.

Solche gemischten Anhydride der Formel III können hergestellt werden, indem man die Carbonsäure II und ein geeignetes Amin in äquimolaren Mengen in einem geeigneten Lösungsmittel löst und mit 1 bis 1,05 Äquivalenten eines Sulfonsäurederivates der Formel VII reagieren läßt.

Als Lösungsmittel eignen sich alle Solventien, die unter den Reaktionsbedingungen stabil sind, wie z.B. Diethylether, Tetrahydrofuran, Acetonitril, Aceton, Methylenchlorid, Chloroform oder Dimethylformamid.

Als Amin eignen sich tertiäre Amine wie z.B. Triethylamin oder Tributylamin aber auch sterisch gehinderte sekundäre Amine wie z.B. Diisopropylamin.

Die Umsetzungen können bei Temperaturen zwischen –80 °C und Raumtemperatur durchgeführt werden, wobei tiefe Temperaturen eine Isomerisierung der Substituenten an der Doppelbindung vermeiden. Vorteilhaft werden die Reaktionen bei –20 bis –50 °C bei einer Reaktionsdauer von 10 min bis 10 h durchgeführt.

Die Verbindungen der Formel III können isoliert werden, indem man z.B. in Tetrahydrofuran als Lösungsmittel und mit Triethylamin als Base arbeitet, das gebildete Triethylaminhydrochlorid absaugt und das Lösungsmittel im Vakuum abdestilliert. Es ist aber zweckmässiger, die erhaltenen Lösungen der Verbindungen der Formel III direkt mit den Cephalosporanaten der Formel IV umzusetzen. Hierzu löst man die Cephalosporanate der Formel IV oder deren Salze in einem geeigneten Lösungsmittel mit 1 bis 4 Äquivalenten eines Amins, kühlt die Lösung auf die gewünschte, anschließende Reaktionstemperatur vor und gibt bei dieser Temperatur diese Lösung zu der oben beschriebenen Lösung der Verbindung der Formel III. Zur Vermeidung einer Isomerisierung des Restes $R^1$ in den Reaktionsprodukten der Formel V führt man die Reaktion zweckmäßigerweise bei –60 bis –30 °C durch und läßt den Ansatz über Nacht auf Raumtemperatur kommen.

Zum Lösen der Verbindungen der Formel IV können die bei der Herstellung der Verbindungen der Formel III genannten Lösungsmittel sowie als Base die dort genannten Amine verwendet werden.

Im allgemeinen ist die Löslichkeit der Verbindungen der Formel IV in diesen Lösemitteln sehr beschränkt, so daß man hier vorteilhaft in an sich bekannter Weise silyliert oder mit Wasser als Lösemittel arbeitet.

Es ist besonders vorteilhaft, die Carbonsäuren VI ohne Schutzgruppe mit den Sulfonsäurederivaten VII in die gemischten Anhydride der Formel VIII zu überführen und diese mit IV direkt zu den Verbindungen der allgemeinen Formel I umzusetzen.

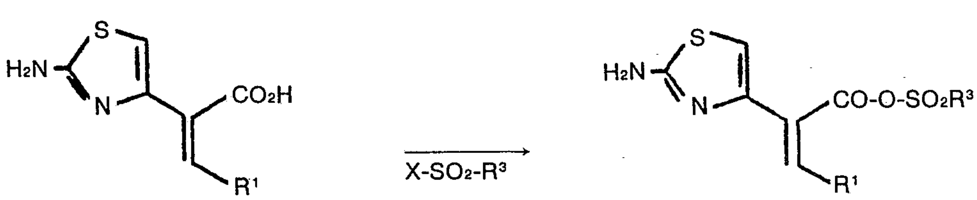


(VI) (VII) (VIII)

$$\text{VII} + \text{IV} \xrightarrow{\text{Base}} \text{I}$$

Es bedeuten

X Cl, Br, $OSO_2R^3$,

$R^3$ einen Alkylrest mit 1 bis 10 C-Atomen, der gegebenenfalls durch Fluor, Chlor, CN, Phenyl, Alkyloxycarbonyl, Alkyloxy oder Alkyl substituiert sein kann, wobei letztere Alkylreste 1 bis 4 C-Atome tragen können, oder ein Phenylrest, der gegebenenfalls durch Fluor, Chlor, Brom, CN, Alkyl, Alkyloxy, Alkylthio, Alkyloxycarbonyl – wobei letztere Alkylgruppen 1 bis 4 C-Atome tragen können – Nitro, Trifluormethyl und Phenyl substituiert sein kann.

Wenn $R^3$ substituiert ist, sind vorzugsweise 1 bis 3 Substituenten, vorzugsweise die genannten vorhanden.

Ganz besonders bevorzugt stellt $R^3$ einen Methyl- oder p-Tolylrest dar.

Die gemischten Anhydride der Formel VIII werden in Analogie zu den Anhydriden der Formel III hergestellt, indem man die Carbonsäuren der Formel VI und 1 bis 1,4 Äquivalente eines Amins in einem Lösungsmittel löst und mit 1 bis 1,2 Äquivalenten eines Sulfonsäurederivates der Formel VII reagieren läßt.

Als Lösungsmittel eignen sich alle Solventien, die unter den Reaktionsbedingungen stabil sind, wie z.B. Diethylether, Tetrahydrofuran, Acetonitril, Aceton, Methylenchlorid, Chloroform oder Dimethylformamid.

Als Amin eignen sich tertiäre Amine wie z.B. Triethylamin oder Tributylamin, aber auch sterisch gehinderte sekundäre Amine wie z.B. Diisopropylamin.

Die Umsetzungen können bei Temperaturen zwischen –80 °C und Raumtemperatur durchgeführt werden, wobei tiefe Temperaturen eine Isomerisierung der Substituenten an der Doppelbindung vermeiden. Vorteilhaft wird die Umsetzung mit $Cl-SO_2CH_3$, in Dimethylformamid bei –40 bis –60 °C durchgeführt.

Zum Lösen der Verbindungen der Formel IV können die bei der Herstellung der Verbindungen der Formel VIII genannten Lösungsmittel sowie als Base die dort genannten Amine verwendet werden.

Im allgemeinen ist die Löslichkeit der Verbindungen der Formel IV in diesen Lösungsmitteln sehr beschränkt, so daß man hier vorteilhaft in an sich bekannter Weise silyliert oder mit Wasser als Lösungsmittel arbeitet.

Die Verbindungen der Formel VI erhält man, indem man von den Verbindungen der Formel II die Schutzgruppe $R^3$ abspaltet – z.B. die Boc-Schutzgruppe mit Trifluoressigsäure.

Ein weiteres Verfahren zur Herstellung der Verbindungen der allgemeinen Formel I ist die Umsetzung von Cephalosporinen der Formel XV mit ungeladenen stickstoffhaltigen Heterocyclen A', wobei A' und $R^1$ die obengenannte Bedeutung aufweisen.

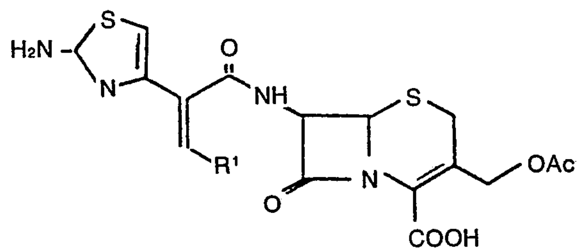 + A-H → I (XV)

Die Umsetzung wird in einem polaren organischen Lösungsmittel, wie Dimethylformamid, oder vorzugsweise in Wasser oder in einem Gemisch aus Wasser und einem mit Wasser leicht mischbaren organischen Lösungsmittel, wie z.B. Aceton, Dioxan, Acetonitril, Dimethylformamid, Dimethylsulfoxid oder Ethanol, durchgeführt. Die Reaktionstemperatur liegt im allgemeinen im Bereich von etwa 10 bis etwa 100 °C, vorzugsweise zwischen 20 und 80 °C. Die Pyridinkomponente A' wird in Mengen zugegeben, die zwischen etwa äquimolaren Mengen und einem bis zu etwa 5fachen Überschuß liegen. Der Austausch wird durch Anwesenheit von Neutralsalzionen, vorzugsweise von Iodid- oder Thiocyanationen, im Reaktionsmedium erleichtert. Insbesondere werden etwa 10 bis etwa 30 Äquivalente Kaliumiodid, Natriumiodid, Kaliumthiocyanat oder Natriumthiocyanat zugegeben. Die Reaktion wird vorteilhaft in der Nähe des Neutralpunktes, vorzugsweise bei einem pH-Wert im Bereich von etwa 5 bis etwa 8 durchgeführt.

Zur Isolierung der Produkte der allgemeinen Formel I ist es vorteilhaft, nach Extraktion des Pyridins A' das erhaltene Rohprodukt an einem Harz wie z.B. Diaion HP 20 oder XAD 7 oder auch an Cellulose zu chromatographieren.

Aus den Verbindungen der Formel XV können die erfindungsgemäßen Verbindungen der allgemeinen Formel I besonders bevorzugt auch dadurch hergestellt werden, daß in literaturbekannter Weise (Tetrahedron Letters, 1981, 3915) in einem organischen Lösungsmittel mit Trimethylsilyliodid intermediär ein reaktives Iodid erzeugt wird, welches dann mit ungeladenen heterocyclischen Basen A' zu Verbindungen der allgemeinen Formel I reagiert.

Die Verbindungen der Formel XV können erhalten werden in analoger Weise wie die erfindungsgemäßen Verbindungen der allgemeinen Formel I. Hierzu wird lediglich bei der Kupplung der Verbindungen der Formel VIII statt des Cephalosporanats der Formel IV 7-Aminocephalosporansäure eingesetzt.

Die erfindungsgemäßen Verbindungen weisen eine starke und breite antimikrobielle Wirksamkeit besonders gegen gramnegative und grampositive Bakterien auf. Diese Eigenschaften ermöglichen ihre Verwendung als chemotherapeutische Wirkstoffe in der Medizin. Mit ihrer Hilfe können die durch gram negative und gram positive Bakterien und bakterienähnliche Mikroorganismen hervorgerufenen Erkrankungen verhindert, gebessert und/oder geheilt werden.

Besonders wirksam sind die erfindungsgemäßen Verbindungen gegen Bakterien und bakterienähnliche Mikroorganismen.

Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staphylococcus aureus, Staph. epidermidis, Staph. aerogenes und Gaffkya tetragena (Staph. = Staphylococcus);

Lactobacteriaceae, wie Streptokokken, z.B. Streptococcus pyogenes, α- bzw. β-hämolysierende Streptokokken, nicht (γ-)-hämolysierende Streptokokken, Str. viridans, Str. faecalis (Enterokokken) und Dipolococcus pneumoniae (Pneumokokken) (Str. = Streptococcus);

Enterobacteriaceae, wie Escherichiae-Bakterien der Coli-Grupe: Escherichia-Bakterien, z.B. Escherichia coli, Enterobacter-Bakterien, z.B. E. aerogenes, E. cloacae, Klebsiella-Bakterien, z.B. K. pneumoniae, Serratia, z.B. Serratia marvescens (E. = Enterobacter) (K. = Klebsiella), Proteae-Bakterien der Proteus-Gruppe: Proteus, z.B. Proteus vulgaris, Pr. morganii, Pr. rettgeri, Pr. mirabillis (Pr. = Proteus);

Pseudomonadaceae, wie Pseudomonas-Bakterien, z.B. Pseudomonas aeruginosa;

Bacteroidaceae, wie Bacteroides-Bakterien, z.B. Bacteroides fragilis.

Die obige Aufzählung von Erregern ist lediglich beispielhaft und keineswegs beschränkend aufzufassen.

Als Krankheiten, die durch die erfindungsgemäßen Verbindungen verhindert, gebessert und/oder geheilt werden können seien beispielsweise genannt:

Erkrankungen der Atemwege und des Rachenraumes;

Otitis; Pharyngitis; Pneumonie; Peritonitis; Pyelonephritis; Cystitis; Endocarditis; Systeminfektionen; Bronchitis; Arthritis; lokale Infektionen.

Zur Erfindung gehören pharmazeutische Zubereitungen, die neben nichttoxischen, inerten pharmazeutisch geeigneten Trägerstoffen eine oder mehrere erfindungsgemäße Verbindungen enthalten oder die aus einem oder mehreren erfindungsgemässen Werkstoffen bestehen.

Zur Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder ½, ⅓ oder ¼ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien und Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzukker, Glucose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium-Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette oder Gemische dieser Stoffe.

Zur parenteralen Applikation können die Lösungen auch in steriler und blutisotonischer Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitonal und/oder rectal, vorzugsweise oral oder parenteral wie intravenös oder intramuskulär appliziert werden.

Im allgemeinen hat es sich sowohl in der Humanals auch in der Veterinärmedizin als vorteilhaft erwiesen, den oder die erfindungsgemäßen Wirkstoffe in Gesamtmengen von etwa 1 bis etwa 1000, vorzugsweise 1 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 1 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die erfindungsgemäßen Verbindungen können zum Zwecke der Erweiterung des Wirkungsspektrums mit einem anderen β-Lactamantibiotikum oder auch mit Aminoglykosidantibiotica, wie z.B. Gentamicin, Sisomicin, Kanamicin, Amikacin oder Tobramicin kombiniert werden.

Die erfindungsgemäßen Verbindungen eignen sich besonders zur Verwendung bei der Bekämpfung von Infektionskrankheiten, insbesondere bei der Bekämpfung bakterieller Infektionen.

Beispiel 1
7-Amino-3-(2-hydroxymethylpyridinium)methyl-3-cephem-4-carboxylat

16 g 7-β-Phenacetylamido-3-cephem-4-carbonsäure wurden durch Zugabe von 3,8 g Natriumhydrogencarbonat in 16 ml Wasser gelöst. Nach Zugabe von 72 g Kaliumrhodanid und 4,8 g 2-Hydroxymethylpyridin wurde 5 h bei 60 °C gerührt. Die Reaktionslösung wurde mit 600 ml Wasser verdünnt und dann im Eisbad gekühlt. Durch langsames Zutropfen von 0,5 N-Salzsäure wurde auf pH 2 angesäuert. Nach 3 h wurde der entstandene Niederschlag abgesaugt, mit kaltem Wasser gewaschen, in 100 ml Wasser aufgeschlämmt und durch Neutralisieren mit Triethylamin auf pH 7,8 gelöst. Durch Zugabe von 8 g Penicillin-Acylase-Harz wurde dann bei pH 7,8 enzymatisch die Phenacetylschutzgruppe abgespalten. Nach Beendigung der Spaltung wurde vom Enzymharz abfiltriert, mit konz. Salzäure auf pH 1 angesäuert und dann mit Aceton das Hydrochlorid der Titelverbindung ausgefällt.

Ausbeute: 1,6 g.

$^1$H-NMR ($D_2O$) δ (ppm) = 8,71 (1H, d, J = 7, H-6-Py); 8,45 (1H, m, H-4-Py); 8,13 (1H, m, H-3-Py); 7,87 (1H, m, H-5-Py); 5,43 (2H, m, $CH_2$-Py); 5,25 (1H, d, J = 6 Hz, H-7-Lactam); 5,13 (1H, d, J = 6 Hz; Z-6-Lactam); 4,94 (2H, bs, Py-$CH_2$–OH); 3,55 (1H, d, J = 18 Hz, S-$CH_2$); 3,28 (1H, d, J = 18 Hz, S-$CH_2$).

Beispiel 2
7-Amino-3-(3-hydroxymethylpyridinium)methyl-3-cephem-4-carboxylat

Die Herstellung erfolgte analog Beispiel 1.

$^1$H-NMR ($D_2O$) δ (ppm) = 8,85 (1H, s, H-2-Py); 9,78 (1H, d, J = 7 Hz, H-6-Py); 8,45 (1H, d, J = 8 Hz, H-4-Py); 7,99 (1H, dd, J = 7 Hz, J = 8 Hz, H-5-Py); 5,54 (1H, d, J = 15 Hz, $CH_2$-Py); 5,31 (1H, d, J = 15 Hz, $CH_2$-Py); 5,26 (1H, d, J = 5 Hz, H-7-Lactam); 5,13 (1H, d, J = 5 Hz, H-6-Lactam); 4,80 (2H, s, Py-$CH_2$–OH); 3,63 (1H, d, J = 18 Hz, S-$CH_2$); 3,37 (1H, d, J = 18 Hz, S-$CH_2$).

Beispiel 3
7-Amino-3-(4-hydroxymethylpyridinium)methyl-3-cephem-4-carboxylat

Die Herstellung erfolgte analog Beispiel 1.

$^1$H-NMR ($D_2O$) δ (ppm) = 8,84 (2H, d, J = 8 Hz, H-2,6-Py); 8,02 (2H, d, J = 8 Hz, H-3,5-Py); 5,62 (1H, d, J = 15 Hz, $CH_2$-Py); 5,39 (1H, d, J = 15 Hz, $CH_2$-Py); 5,37 (1H, d, J = 5 Hz, H-7-Lacatam); 5,23 (1H, d, J = 5 Hz, H-6-Lactam); 4,98 (2H, s, Py-$CH_2$-OH); 3,74 (1H, d, H = 18 Hz, S-$CH_2$); 3,40 (1H, d, J = 18 Hz, S-$CH_2$).

Beispiel 4
7-Amino-3-(3-caboxymethylpyridinium)methyl-3-cephem-4-carboxylat

Die Herstellung erfolgte analog Beispiel 1.

$^1$H-NMR ($D_2O$) δ (ppm) = 8,85 (1H, s, H-2-Py); 8,80 (1H, d, J = 7 Hz, H-6-Py); 8,43 (1H, d, J = 8 Hz, H-4-Py); 7,99 (1H, m, H-5-Py); 5,54 (1H, d, J = 14 Hz, $CH_2$-Py); 5,28 (1H, d, J = 14 Hz, $CH_2$-Py); 5,23 (1H, d, J = 5 Hz, H-7-Lactam); 5,11 (1H, d, J = 5 Hz, H-6-Lactam); 3,94 (2H, bs, Py-$CH_2$–); 3,60 (1H, d, J = 18 Hz, S-$CH_2$); 3,24 (1H, d, J = 18 Hz, S-$CH_2$).

Beispiel 5
7-Amino-3-(4-carboxypyridinium)methyl-3-cephem-4-carboxylat

Die Herstellung erfolgte analog Beispiel 1.

$^1$H-NMR ($D_2O$) δ (ppm) = 9,03 (2H, d, J = 7 Hz, H-2,6-Py); 8,38 (2H, d, J = 7 Hz, H-3,5-Py); 5,74 (1H, d, J = 15 Hz, $CH_2$-Py); 5,41 (1H, d, J = 15 Hz, $CH_2$-Py); 5,31 (1H, d, J = 5 Hz, H-7-Lactam); 5,19 (1H, d, J = 5 Hz, H-6-Lactam); 3,72 (1H, d, J = 18 Hz, S-$CH_2$); 3,37 (1H, d, J = 18 Hz, S-$CH_2$).

Beispiel 6
7-Amino-3-(4-cyclopropylpyridinium)methyl-3-cephem-4-carboxylat

Die Herstellung erfolgte analog Beispiel 1.

$^1$H-NMR ($D_2O$) δ (ppm) = 8,55 (2H, d J = 7 Hz, H-2,6-Py); 7,60 (2H, d, J = 7 Hz, H-3,5-Py); 5,43 (1H, d, J = 15 Hz, $CH_2$-Py); 5,27 (1H, d, J = 5 Hz, H-7-Lactam); 5,25 (1H, d, J = 15 Hz, $CH_2$-Py); 3,60 (1H, d, J = 18 Hz, S-$CH_2$); 3,28 (1H, d, J = 18 Hz, S-$CH_2$); 2,18 (1H, m, Cycloprop.); 1,40 (2H, m, Cycloprop.); 1,08 (2H, m, Cycloprop.).

Beispiel 7
7-Amino-3-(4-benzylpyridinium)methyl-3-cephem-4-carboxylat

Die Herstellung erfolgte analog Beispiel 1.

$^1$H-NMR (DMSO) δ (ppm) = 9,06 (2H, d, J = 7 Hz,

H-2,6-Py); 8,11 (2H, d, J = 7 Hz, H-3,5-Py); 7,20–7,30 (5H, m, arom.); 5,59 (1H, d, J = 13 Hz, $CH_2$-Py); 5,50 (1H, d, J = 13 Hz, $CH_2$-Py); 5,15 (1H, d, J = 5 Hz, H-7-Lactam); 5,03 (1H, d, J = 5 Hz, H-6-Lactam); 4,31 (2H, bs, $CH_2$-Φ); 3,60 (1H, d, J = 18 Hz, S-$CH_2$); 3,46 (1H, d, J = 18 Hz, S-$CH_2$).

Beispiel 8

7-Amino-3-(4-phenylpyridinium)methyl-3-cephem-4-carboxylat

Die Herstellung erfolgte analog Beispiel 1.

$^1$H-NMR ($D_2O$) δ (ppm) = 8,78 (2H, d, J = 7 Hz, H-2,6-Py); 8,18 (2H, d, J = 7 Hz, H-3,5-Py); 7,81 (2H, m, arom.); 7,52 (3H, m, arom.); 5,42 (1H, d, J = 15 Hz, $CH_2$-Py); 5,22 (1H, d, J = 15 Hz, $CH_2$-Py); 5,20 (1H, d, J = 5 Hz, H-7-Lactam); 5,09 (1H, d, J = 5 Hz, H-6-Py); 3,58 (1H, d, J = 18 Hz, S-$CH_2$); 3,23 (1H, d, J = 18 Hz, S-$CH_2$).

Beispiel 9

7-Amino-3-(2,3-cyclopentenopyridinium)methyl-3-cephem-4-carboxylat

Die Herstellung erfolgt analog Beispiel 1.

$^1$H-NMR ($D_2O$) δ (ppm) = 8,56 (1H, d, J = 7 Hz, H-6-Py); 8,35 (1H, d, J = 8 Hz, H-4-Py); 7,82 (1H, m, H-5-Py); 5,61 (1H, d, J = 15 Hz, $CH_2$-Py); 5,53 (1H, d, J = 15 Hz, $CH_2$-Py); 5,39 (1H, d, J = 5 Hz, H-7-Lactam); 5,27 (1H, d, J = 5 Hz, H-6-Lactam); 3,66 (1H, d, J = 18 Hz, S-$CH_2$); 3,45 (1H, d, J = 18 Hz, S-$CH_2$); 3,38 (2H, m, Cyclopent.); 3,23 (2H, m, Cyclopent.); 2,35 (2H, m, Cyclopent.).

Beispiel 10

7-Amino-3-(5,6,7,8-tetrahydrochinolinium)methyl-3-cephem-4-carboxylat

Die Herstellung erfolgte analog Beispiel 1.

$^1$H-NMR ($D_2O$) δ (ppm) = 8,49 (1H, d, J = 7 Hz, H-6-Py); 8,26 (1H, d, J = 8 Hz, H-4-Py); 7,68 (1H, m, H-5-Py); 5,50 (1H, d, J = 15 Hz, $CH_2$-Py); 5,41 (1H, d, J = 15 Hz, $CH_2$-Py); 5,27 (1H, d, J = 5 Hz, H-7-Lactam); 5,14 (1H, d, J = 5 Hz, H-6-Lactam); 3,47 (1H, d, J = 18 Hz, S-$CH_2$); 3,32 (1H, d, J = 18 Hz, S-$CH_2$); 2,97 (2H, m, Cyclohex.); 2,89 (2H, m, Cyclohex.); 1,86 (2H, m, Cyclohex.); 1,73 (2H, m, Cyclohex.).

Beispiel 11

7-Amino-3-isochinoliniummethyl-3-cephem-4-carboxylat

Die Herstellung erfolgte analog Beispiel 1.

$^1$H-NMR ($D_2O$) δ (ppm) = 9,74 (1H, s, H-1-Isoch.); 8,57 (1H, d, J = 7 Hz, H-3-Isoch.); 8,39 (2H, m, Isoch.); 8,20 (2H, m, Isoch.); 8,01 (1H, m, Isoch.); 5,72 (1H, d, J = 15 Hz, $CH_2$-Isoch.); 5,49 (1H, d, J = 15 Hz, $CH_2$-Isoch.); 5,29 (1H, d, J = 5 Hz, H-7-Lactam); 5,16 (1H, d, J = 5 Hz, H-6-Lactam); 3,68 (1H, d, J = 18 Hz, S-$CH_2$); 3,45 (1H, d, J = 18 Hz, S-$CH_2$).

Beispiel 12

7-Amino-3-chinoliniummethyl-3-cephem-4-carboxylat

Die Herstellung erfolgte analog Beispiel 1.

$^1$H-NMR ($D_2O$/DMSO-$d_6$) δ (ppm) = 9,24 (1H, d, J = 7 Hz, H-2-Chin.); 9,16 (1H, d, J = 8 Hz, H-4-Chin.); 8,40 (2H, m. Chin.); 8,22 (1H, m, Chin.); 8,04 (2H, m, Chin.); 6,00 (1H, d, J = 15 Hz, $CH_2$-Chin.); 5,85 (1H, d, J = 15 Hz, $CH_2$-Chin.); 5,21 (1H, d, J = 5 Hz, H-7-Lactam); 5,12 (1H, d, J = 5 Hz, H-6-Lactam); 3,44 (1H, d, J = 18 Hz, S-$CH_2$); 3,24 (1H, d, J = 18 Hz, S-$CH_2$).

Beispiel 13

7-Amino-3-(4-methoxymethylpyridinium)methyl-3-cephem-4-carboxylat

Die Herstellung erfolgte analog Beispiel 1.

$^1$H-NMR ($D_2O$) δ (ppm) = 8,85 (2H, d, J = 7 Hz, H-2,6-Py); 8,00 (2H, d, J = 7 Hz, H-3,5-Py); 5,61 (1H, d, J = 15 Hz, $CH_2$-Py); 5,33 (1H, d, J = 15 Hz, $CH_2$-Py); 5,29 (1H, d, J = 5 Hz, H-7-Lactam); 5,19 (1H, d, J = 5 Hz, H-6-Lactam); 4,80 (2H, bs, Py-$CH_2$); 3,58 (1H, d, J = 18 Hz, S-$CH_2$); 3,47 (3H, s, $OCH_3$).

Beispiel 14

7-Amino-3-(4-methoxypyridinium)methyl-3-cephem-4-carboxylat

Die Herstellung erfolgte analog Beispiel 1.

$^1$H-NMR ($D_2O$) δ (ppm) = 8,65 (2H, d, J = 7 Hz, H-2,6-Py); 7,45 (2H, d, J = 7 Hz, H-3,5-Py); 5,40 (1H, d, J = 15 Hz, $CH_2$-Py); 5,27 (1H, d, J = 5 Hz, H-7-Lactam); 5,18 (2H, m, $CH_2$-Py, H-6-Lactam); 4,07 (3H, s, $OCH_3$); 3,63 (1H, d, J = 18 Hz, S-$CH_2$); 3,30 (1H, d, J = 18 Hz, S-$CH_2$).

Beispiel 15

7-[1-(2-Aminothiazol-4-yl)-1(Z)-propencarboxamido]-3-(4-hydroxymethylpyridinium)-methyl-3-cephem-4-carboxylat

Unter Stickstoff wurden 736 mg (4 mmol) 1-(2-Aminothiazol-4-yl)-1(Z)-propencarbonsäure in 5 ml absol. DMF gelöst und mit 767 µl (4.4 mmol) Ethyldiisopropylamin versetzt. Nach Abkühlen auf –55 °C wurde mit 320 µl Methansulfonsäurechlorid versetzt und 40 min nachgerührt. 1,50 g (4 mmol) 7-Amino-3-(4-hydroxymethylpyridinium)methyl-3-cephem-4-carbonsäurechlorid-hydrat wurden inzwischen in 2 ml Wasser gelöst, durch Zugabe von Triethylamin auf pH 7 gestellt und auf 10 °C gekühlt. Anschließend wurden nochmal 2 ml Triethylamin zugegeben, und dann wurde die –55 °C kalte DMF-Lösung unter Rühren schnell zugegossen. Nach 10 min wurde die Reaktionslösung auf 600 ml Aceton gegeben, der entstehende Niederschlag wurde abgesaugt, in wenig Wasser gelöst und über Adsorberharz HP 20 gereinigt (Elution mit Waser und steigenden Mengen Acetonitril). Abschließend wurde lyophilisiert.

Ausbeute: 650 mg.

$^1$H-NMR ($D_6$-DMSO) δ (ppm) = 9,35 (2H, d, J = 7 Hz, H-2,6-Py); 9,18 (1H, d, J = 9 Hz, NH); 8,03 (2H, d, J = 7 Hz, H-3,5-Py); 6,98 (2H, bs, $NH_2$); 6,38 (1H, q, J = 8 Hz, C=CH); 6,17 (1H, s,Thiazol); 5,67 (1H, dd, J = 5 Hz, J = 9 Hz, H-7-Lactam); 5,62 (1H, d, J = 14 Hz, $CH_2$-Py); 5,09 (1H, d, J = 5 Hz, H-6-Lactam); 5,08 (1H, d, J = 14 Hz, $CH_2$-Py); 4,79 (2H, s, Py-$CH_2$); 3,54 (1H, d, J = 18 Hz, S-$CH_2$); 3,06 (1H, d, J = 18 Hz, S-$CH_2$); 1,75 (3H, d, J = 8 Hz, C=C–$CH_3$).

Beispiel 16

7-[1-(2-Aminothiazol-4-yl)-1(Z)-propencarboxamido]-3-(3-hydroxymethylpyridinium)methyl-3-cephem-4-carboxylat

Die Herstellung erfolgte analog Beispiel 15.

$^{1}H$-NMR ($D_6$-DMSO) δ (ppm) = 9,47 (1H, d, J = 7 Hz, H-6-Py); 9,35 (1H, s, H-2-Py); 9,23 (1H, d, J = 9 Hz, NH); 8,53 (1H, d, J = 8 Hz, H-4-Py); 8,18 (1H, m, H-5-Py); 7,01 (2H, bs, $NH_2$); 6,32 (1H, q, J = 8 Hz, C = CH); 6,20 (1H, s, Thiazol); 5,72 (2H, m, $CH_2$-Py, H-7-Lactam); 5,18 (1H, d, J = 18 Hz, $CH_2$-Py); 5,13 (1H, d, J = 5 Hz, H-6-Lactam); 4,74 (2H, s, Py-$CH_2$); 3,56 (1H, d, J = 18 Hz, S-$CH_2$); 3,12 (1H, d, J = 18 Hz, S-$CH_2$); 1,77 (3H, d, J = 8 Hz, C = C–$CH_3$).

Beispiel 17

7-[1-(2-Aminothiazol-4-yl)-1(Z)-propencarbox-amido]-3-(2-hydroxymethylpyridinium)methyl-3-cephem-4-carboxylat

Die Herstellung erfolgte analog Beispiel 15.

$^{1}H$-NMR ($D_6$-DMSO) δ (ppm) = 9,37 (1H, d, J = 7 Hz, H-6-Py); 9,23 (1H, d, J = 9 Hz, NH); 8,58 (1H, m H-4-Py); 8,19 (1H, d, J = 8 Hz, H-3-Py); 8,08 (1H, m, H-5-Py); 7,00 (2H, bs, $NH_2$); 6,32 (1H, q J = 8 Hz, C = CH); 6,20 (1H, s, Thiazol); 5,71 (1H, dd, J = 5 Hz, J = 9 Hz, H-7-Lactam); 5,46 (1H, d, J = 14 Hz, $CH_2$-Py); 5,31 (1H, d, J = 14 Hz, $CH_2$-Py); 5,11 (1H, d, J = 5 Hz, H-6-Lactam); 4,98 (2H, s, Py-$CH_2$); 3,46 (1H, d, J = 18 Hz, S-$CH_2$); 3,08 (1H, d, J = 18 Hz, S-$CH_2$); 1,78 (3H, d, J = 8 Hz, C = C–$CH_3$).

Beispiel 18

7-[1-(2-Aminothiazol-4-yl)-1(Z)-propencarbox-amido]-3-[4-(2-sulfoethyl)pyridinium]methyl-3-cephem-4-carboxylat

Die Herstellung erfolgte analog Beispiel 15.

$^{1}H$-NMR ($D_6$-DMSO) δ (ppm) = 9,32 (2H, d, J = 7 Hz, H-2,6-Py); 9,20 (1H, d, J = 9 Hz, NH); 8,06 (2H, d, J = 7 Hz, H-3,5-Py); 6,97 (2H, bs, $NH_2$); 6,28 (1H, q, J = 8 Hz, C = CH); 6,17 (1H, s, Thiazol); 5,67 (1H, dd, J = 5 Hz, J = 9 Hz, H-7-Lactam); 5,61 (1H, d, J = 14 Hz, $CH_2$-Py); 5,10 (1H, d, J = 5 Hz, H-6-Lactam); 4,97 (1H, d, J = 14 Hz, $CH_2$-Py); 3,53 (1H, d, J = 18 Hz, S-$CH_2$); 3,17 (2H, t, J = 8, Py-$CH_2$-$CH_2$); 3,02 (1H, d, J = 18 Hz, S-$CH_2$); 2,84 (2H, t, J = 8 Hz, Py-$CH_2$-$CH_2$); 1,75 (3H, d, J = 8 Hz, C = C–$CH_3$).

Beispiel 19

7-[1-(2-Aminothiazol-4-yl)-1(Z)-propencarbox-amido]-3-(4-carboxypyridinium)methyl-3-cephem-4-carboxylat

Die Herstellung erfolgte analog Beispiel 15.

$^{1}H$-NMR ($D_6$-DMSO) δ (ppm) = 9,19 (2H, d, J = 7 Hz, H-2,6-Py); 9,12 (1H, d, J = 9 Hz, NH); 8,15 (2H, d, J = 7 Hz, H-3,5-Py); 6,91 (2H, bs, $NH_2$); 6,23 (1H, q, J = 8 Hz, C = CH); 6,12 (1H, s, Thiazol); 5,65 (1H, dd, J = 5 Hz, J = 9 Hz, H-7-Lactam); 5,57 (1H, d, J = 13 Hz, $CH_2$-Py); 5,10 (1H, d, J = 13 Hz, $CH_2$-Py); 5,06 (1H, d, J = 5 Hz, H-6-Lactam); 3,48 (1H, d, J = 18 Hz, S-$CH_2$); 3,10 (1H, d, J = 18 Hz, S-$CH_2$); 1,73 (3H, d, J = 8 Hz, C = C–$CH_3$).

Beispiel 20

7-[1-(2-Aminothiazol-4-yl)-1(Z)-propencarbox-amido]-3-(3-carboxypyridinium)methyl-3-cephem-4-carboxylat

Die Herstellung erfolgte analog Beispiel 15.

$^{1}H$-NMR ($D_6$-DMSO) δ (ppm) = 9,48 (1H, s, H-2-Py); 9,42 (1H, d, J = 7 Hz, H-6-Py); 9,21 (1H, d, J = 9 Hz, NH); 8,75 (1H, d, J = 8 Hz, H-4-Py); 8,05 (1H, m, H-5-Py); 6,95 (2H, bs, $NH_2$); 6,29 (1H, q, J = 8 Hz, C = CH); 6,17 (1H, s, Thiazol); 5,70 (2H, m, $CH_2$-Py, H-7-Lactam); 5,18 (1H, d, J = 14 Hz, $CH_2$-Py); 5,09 (1H, d, J = 5 Hz, H-6-Lactam); 3,51 (1H d, J = 18 Hz, S-$CH_2$); 3,03 (1H, d, J = 18 Hz, S-$CH_2$); 1,76 (3H, d, J = 8 Hz, C = C–$CH_3$).

Beispiel 21

7-[1-(2-Aminothiazol-4-yl)-1(Z)-propencarbox-amido]-3-(3-carboxymethylpyridinium)methyl-3-cephem-4-carboxylat

Die Herstellung erfolgte analog Beispiel 15.

$^{1}H$-NMR ($D_6$-DMSO) δ (ppm) = 9,43 (1H, d, J = 7 Hz, H-6-Py); 9,27 (1H, s, H-2-Py); 9,21 (1H, d, J = 9 Hz, NH); 8,46 (1H, d, J = 8 Hz, H-4-Py); 8,09 (1H, m, H-5-Py); 6,99 (2H, bs, $NH_2$); 6,30 (1H, q, J = 8 Hz, C = CH); 6,18 (1H, s, Thiazol); 5,70 (2H, m, $CH_2$-Py, H-7-Lactam); 5,12 (2H, m, $CH_2$-PY, H-6-Lactam); 3,69 (2H, bs, Py-$CH_2$); 3,56 (1H, d, J = 18 Hz, S-$CH_2$); 3,09 (1H, d, J = 18 Hz, S-$CH_2$); 1,76 (3H, d, J = 8 Hz, C = C–$CH_3$).

Beispiel 22

7-[1-(2-Aminothiazol-4-yl)-1(Z)-propencarbox-amido]-3-(4-cyclopropylpyridinium)methyl-3-cephem-4-carboxylat

Die Herstellung erfolgte analog Beispiel 15.

$^{1}H$-NMR ($D_6$-DMSO) δ (ppm) = 9,21 (2H, d, J = 7 Hz, H-2,6-Py); 9,17 (1H, d, J = 9 Hz, NH); 7,82 (2H, d, J = 7 Hz, H-3,5-Py); 6,98 (2H, bs, $NH_2$); 6,20 (1H, q, J = 8 Hz, C = CH); 6,18 (1H, s, Thiazol); 5,67 (1H, dd, J = 5 Hz, J = 9 Hz, H-7-Lactam); 5,55 (1H, d, J = 15 Hz, $CH_2$-Py); 5,09 (1H, d, J = 5 Hz, H-6-Lactam); 4,98 (1H, d, J = 15 Hz, $CH_2$-Py); 3,53 (1H, d, J = 18 Hz, S-$CH_2$); 3,04 (1H, d, J = 18 Hz, S-$CH_2$); 2,28 (1H, m, Cycloprop.); 1,77 (3H, d, J = 8 Hz, C = C–$CH_3$); 1,39 (2H, m, Cycloprop.); 1,14 (2H, m, Cycloprop.).

Beispiel 23

7-[1-(2-Aminothiazol-4-yl)-1(Z)-propencarbox-amido]-3-(4-phenylpyridinium)methyl-3-cephem-4-carboxylat

Die Herstellung erfolgte analog Beispiel15.

$^{1}H$-NMR ($D_6$-DMSO) δ (ppm) = 9,51 (2H, d, J = 7 Hz, H-2,6-Py); 9,18 (1H, d, J = 9 Hz, NH); 8,52 (2H, d, J = 7 Hz, H-3,5-Py); 8,06 (2H, m, arom.); 7,65 (3H, m, arom.); 6,96 (2H, bs, $NH_2$); 6,28 (1H, q, J = 8 Hz, C = CH); 6,17 (1H, s, Thiazol); 5,65 (2H, m, $CH_2$-Py, H-7-Lactam); 5,11 (1H, d, J = 5 Hz, H-6-Lactam); 5,06 (1H, d, J = 14 Hz, $CH_2$-Py); 3,56 (1H, d, J = 18 Hz, S-$CH_2$); 3,11 (1H, d, J = 18 Hz, S-$CH_2$); 1,75 (3H, d, J = 8 Hz, C = C–$CH_3$).

Beispiel 24

7-[1-(2-Aminothiazol-4-yl)-1(Z)-propencarbox-amido]-3-(4-benzylpyridinium)methyl-3-cephem-4-carboxylat

Die Herstellung erfolgte analog Beispiel 15.

$^{1}H$-NMR ($D_6$-DMSO) δ (ppm) = 9,33 (2H, d, J = 7 Hz, H-2,6-Py); 9,14 (1H, d, J = 9 Hz, NH); 8,00 (2H, d, J = 7 Hz, H-3,5-Py); 7,20–7,40 (5H, m, arom.); 6,97 (1H, s, Thiazol); 6,27 (1H, q, J = 8 Hz, C = CH);

6,15 (1H, s, Thiazol); 5,65 (1H, dd, J = 5 Hz, J = 9 Hz, H-7-Lactam); 5,56 (1H, d, J = 13 Hz, $CH_2$-Py); 5,07 (1H, d, J = 5 Hz, H-6-Lactam); 4,99 (1H, d, J = 13 Hz, $CH_2$-Py); 4,22 (2H, bs, Py-$CH_2$-Φ); 3,50 (1H, d, J = 18 Hz, S-$CH_2$); 3,03 (1H, d, J = 18 Hz, S-$CH_2$); 1,73 (3H, d, J = 8 Hz, C = C–$CH_3$).

Beispiel 25
7-[1-(2-Aminothiazol-4-yl)-1(Z)-propencarbox-amido]-3-(2,3-cyclopentenopyridinium)methyl-3-cephem-4-carboxylat

Die Herstellung erfolgte analog Beispiel 15.

$^1$H-NMR ($D_6$-DMSO) δ (ppm) = 9,31 (1H, d, J = 7 Hz, H-6-Py); 9,22 (1H, d, J = 9 Hz, NH); 8,37 (1H, d, J = 8 Hz, H-4-Py); 7,91 (1H, dd, J = 7 Hz, J = 8 Hz, H-5-Py); 7,01 (2H, bs, $NH_2$); 6,32 (1H, q, J = 8 Hz, C = CH); 6,20 (1H, s, Thiazol); 5,69 (1H, dd, J = 5 Hz, J = 9 Hz, H-7-Lactam); 5,49 (1H, d, J = 13 Hz, $CH_2$-Py); 5,22 (1H, d, J = 13 Hz, $CH_2$-Py); 5,09 (1H, d, J = 5 Hz, H-6-Lactam); 3,42 (3H, m, S-$CH_2$, Cyclopent.); 3,13 (3H, m, S-$CH_2$, Cyclopent.); 2,23 (2H, m, Cyclopent.); 1,78 (3H, d, J = 8 Hz, C = C–$CH_3$).

Beispiel 26
7-[1-(2-Aminothiazol-4-yl)-1(Z)-propencarbox-amido]-3-(5,6,7,8-tetrahydrochinolinium)methyl-3-cephem-4-carboxylat

Die Herstellung erfolgte analog Beispiel 15.

$^1$H-NMR ($D_6$-DMSO) δ (ppm) = 9,22 (2H, m, NH, H-6-Py); 8,29 (1H, d, J = 8 Hz, H-4-Py); 7,91 (1H, m, H-5-Py); 7,00 (2H, bs $NH_2$); 6,32 (1H, q, J = 8 Hz, C = CH); 6,20 (1H, s, Thiazol); 5,70 (1H, dd, J = 5 Hz, J = 9 Hz, H-7-Lactam); 5,40 (2H, m, $CH_2$-Py); 5,11 (1H, d, J = 5 Hz, H-6-Lactam); 3,45 (1H, d, J = 18 Hz, S-$CH_2$); 3,15 (3H, m, 5-$CH_2$-Cyclohex.); 2,95 (2H, m, Cyclohex.); 1,89 (2H, m, Cyclohex.); 1,78 (5H, m, Cyclohex., C = C–$CH_3$).

Beispiel 27
7-[1-(2-Aminothiazol-4-yl)-1(Z)-propencarbox-amido]-3-chinoliniummethyl-3-cephem-4-carboxylat

Die Herstellung erfolgte analog Beispiel 15.

$^1$H-NMR ($D_6$-DMSO) δ (ppm) = 9,73 (1H, d, J = 7, H-2-Chin.); 9,29 (1H, d, J = 8 Hz. Chin.); 9,18 (1H, d, J = 8 Hz, NH); 9,11 (1H, d, J = 8 Hz, Chin.); 8,48 (1H, d, J = 8 Hz, Chin.); 8,23 (2H, m, Chin.); 8,04 (1H, m, Chin.); 6,97 (2H, bs, $NH_2$); 6,29 (1H, q, J = 8 Hz, C = CH); 6,18 (1H, s, Thiazol); 6,05 (1H, d, J = 13 Hz, $CH_2$-Chin.); 5,91 (1H d, J = 13 Hz, $CH_2$-Chin.); 5,66 (1H, dd, J = 8 Hz, J = 5 Hz, H-7-Lactam); 5,07 (1H, d, J = 5 Hz, H-6-Lactam); 3,40 (1H, d, J = 18 Hz, S-$CH_2$); 3,00 (1H, d, J = 18 Hz, S-$CH_2$); 1,76 (3H, d, J = 8 Hz, C = C–$CH_3$).

Beispiel 28
7-[1-(2-Aminothiazol-4-yl)-1(Z)-propencarbox-amido]-3-isochinolinium-3-cephem-4-carboxylat

Die Herstellung erfolgte analog Beispiel 15.

$^1$H-NMR ($D_6$-DMSO) δ (ppm) = 10,26 (1H, s, H-1-Isochin.); 9,42 (1H, d, J = 8 Hz, H-3-Isochin.); 9,19 (1H, d, J = 9 Hz, NH); 8,59 (1H, d, J = 7 Hz, Isochin.); 8,48 (1H, d, J = 8 Hz, Isochin.); 8,29 (2H, m, Isochin.); 8,05 (1H, m, Isoch.); 6,96 (2H, bs, $NH_2$); 6,28 (1H, q, J = 8 Hz, C = CH); 6,16 (1H, s, Thiazol); 5,83 (1H, d, J = 14 Hz, $CH_2$-Isochin.); 5,67 (1H, dd, J = 5 Hz, J = 9 Hz, H-7-Lactam); 5,24 (1H, d, J = 14 Hz, Isoch.); 5,11 (1H, d, J = 5 Hz, H-6-Lactam); 3,56 (1H, d, J = 18 Hz, S-$CH_2$); 3,19 (1H, d, J = 18 Hz, S-$CH_2$); 1,73 (3H, d, J = 8 Hz, C = C–$CH_3$).

Beispiel 29
7-[1-(2-Aminothiazol-4-yl)-1(Z)-propencarbox-amido]-3-(4-methoxymethylpyridinium)methyl-3-cephem-4-carboxylat

Die Herstellung erfolgte analog Beispiel 15.

$^1$H-NMR ($D_6$-DMSO) δ (ppm) = 9,42 (2H, d, J = 7 Hz, H-2,6-Py); 9,16 (1H, d, J = 9 Hz, NH); 8,03 (2H, d, J = 7 Hz, H-3,5-Py); 6,97 (2H, bs, $NH_2$); 6,29 (1H, q, J = 8 Hz, C = CH); 6,18 (1H, s, Thiazol); 5,65 (2H, m, $CH_2$-Py, H-7-Lactam); 5,09 (1H, d, J = 5 Hz, H-6-Lactam); 5,06 (1H, d, J = 14 Hz, $CH_2$-Py); 4,75 (2H, s, Py-$CH_2$); 3,54 (1H, d, J = 18 Hz, S-$CH_2$); 3,43 (3H, s, $OCH_3$); 3,04 (1H, d, J = 18 Hz, S-$CH_2$); 1,76 (3H, d, J = 8 Hz, C = C–$CH_3$).

Beispiel 30
7-[1-(2-Aminothiazol-4-yl)-1(Z)-propencarbox-amido]-3-(4-methoxypyridinium)methyl-3-cephem-4-carboxylat

Die Herstellung erfolgte analog Beispiel 15.

$^1$H-NMR ($D_6$-DMSO) δ (ppm) = 9,30 (2H, d, J = 7 Hz, H-2,6-Py); 9,17 (1H, d, J = 9 Hz, NH); 7,65 (2H, d, J = 7 Hz, H-3,5-Py); 6,97 (2H, bs, $NH_2$); 6,29 (1H, q, J = 8 Hz, C = CH); 6,18 (1H, s, Thiazol); 5,65 (1H, dd, J = 9 Hz, J = 5 Hz, H-7-Lactam); 5,49 (1H, d, J = 14 Hz, $CH_2$-Py); 5,09 (1H, d, J = 5 Hz, H-6-Lactam); 4,87 (1H, d, J = 14 Hz, $CH_2$-Py); 4,08 (3H, s, $OCH_3$); 3,53 (1H, d, J = 18 Hz, S-$CH_2$); 2,98 (1H, d, J = 18 Hz, S-$CH_2$); 1,75 (3H, d, J = 8 Hz, C = C–$CH_3$).

Analog Beispiel 15 wurden folgende Verbindungen hergestellt:

Beispiel 31
7-[1-(2-Aminothiazol-4-yl)-1(Z)-propencarbox-amido]-3-(2,3-dihydroxymethylpyridinium)methyl-3-cephem-4-carboxylat

Beispiel 32
7-[1-(2-Aminothiazol-4-yl)-1(Z)-propencarbox-amido]-3-(2-methyl-3-hydroxymethylpyridinium)-methyl-3-cephem-4-carboxylat

Beispiel 33
7-[1-(2-Aminothiazol-4-yl)-1(Z)-propencarbox-amido]-3-[4-dimethylaminopyridinium]methyl-3-cephem-4-carboxylat

Beispiel 34
7-[1-(2-Aminothiazol-4-yl)-1(Z)-propencarbox-amido]-3-[3-methylisochinolinium]methyl-3-cephem-4-carboxylat

Beispiel 35
7-[1-(2-Aminothiazol-4-yl)-1(Z)-propencarbox-amido]-3-(5-hydroxymethylchinolinium)methyl-3-cephem-4-carboxylat

Beispiel 36

7-[1-(2-Aminothiazol-4-yl)-1(Z)-propencarboxamido]-3-pyridazinium-3-cephem-4-carboxylat

$^1$H-NMR ($D_6$-DMSO) δ (ppm) = 10,41 (1H, d, J = 7 Hz, Pyrid.); 9,67 (1H, d, J = 4 Hz, Pyrid.); 9,18 (1H, d, J = 9 Hz, NH); 8,74 (1H, m, Pyrid.); 8,59 (1H, m, Pyrid.); 6,98 (2H, bs, $HN_2$); 6.38 (1H, q, J = 8 Hz, C = CH); 6,17 (1H, s, Thiazol); 5,89 (1H, d, J = 14 Hz, $CH_2$-Pyrid.); 5,68 (1H, d, J = 9 Hz, J = 5 Hz, H-7-Lactam); 5,37 (1H, d, J = 14 Hz, $CH_2$-Pyrid.); 5,06 (1H, d, J = 5 Hz, H-6-Lactam); 3,60 (1H, d, J = 18 Hz, S-$CH_2$); 3,34 (1H, d, J = 18 Hz, S-$CH_2$); 3,34 (1H, d, J = 18 Hz, S-$CH_2$); 1,73 (3H, d, J = 8 Hz, C = C–$CH_3$).

Beispiel 37

7-[1-(2-Aminothiazol-4-yl)-1(Z)-propencarboxamido]-3-pyrazinium-3-cephem-4-carboxylat

Beispiel 38

7-[Z-2-(2-Aminothiazol-4-yl)-2-(2,3,6-trichlorbenzyliden)acetamido]-3-(3-carboxypyridinium)-methyl-3-cephem-4-carboxylat

Eine Lösung von 1 g Z-2-(2-Aminothiazol-4-yl)-2-(2,3,6-trichlorbenzyliden)essigsäure in 10 ml absolutem Dimethylformamid wird mit 0,4 g 1-Hydroxybenztriazol und 0,6 g Dicyclohexylcarbodiimid versetzt und vier Stunden bei Raumtemperatur gerührt. Man saugt ab und versetzt das Filtrat mit einer Lösung von 1,3 g 7-Amino-3-(3-carboxypyridinium)-methyl-3-cephem-4carbonsäurehydrochlorid-hydrat in 5 ml Wasser, die durch Zugabe von Triethylamin auf pH 7,0 eingestellt wurde. Nach zweistündigem Rühren bei Raumtemperatur wird 900 ml Aceton eingerührt und das ausgefallene Produkt abgesaugt.

Ausbeute: 1,2 g.

$^1$H-NMR ($D_6$-DMSO) δ (ppm) = 9,62 (1) bs, 9,40 (1) d, J = 6 Hz, 9,10 (1) d, J = 7 Hz, 8,80 (1) d, J = 7 Hz, 8,11 (1) dd, J = 7 Hz, J = 6 Hz, 7,55 (1) d, J = 9 Hz, 7,45 (1) d, J = 9 Hz, 7,28 (2) bs, 7,17 (1) s, 6,62 (1) s, 5,71 (1) d, J = 13 Hz, 5,60 (1) dd, J = 7 Hz, J = 5 Hz, 5,31 (1) d, J = 13 Hz, 5,00 (1) d, J = 5 Hz, 3,23 (1) d, J = 17 Hz, 3,03 (1) d, J = 17 Hz.

Beispiel 39

7-[Z-2-(2-Aminothiazol-4-yl)-(2,3,6-trichlorbenzyliden)acetamido]-3-(4-carboxypyridinium)-methyl-3-cephem-4-carboxylat

Die Herstellung erfolgte analog Beispiel 38.

Beispiel 40

7-[Z-2-(2-Aminothiazol-4-yl)-2-(2,3,6-trichlorbenzyliden)acetamido]-3-(3-carboxymethylpyridinium)methyl-3-cephem-4-carboxylat

Die Herstellung erfolgte analog Beispiel 38.

Beispiel 41

7-[Z-2-(2-Aminothiazol-4-yl)-2-benzylidenacetamido]-3-(3-carboxypyridinium)methyl-3-cephem-4-carboxylat

Die Herstellung erfolgte analog Beispiel 38 Z-2-(2-Aminothiazol-4-yl)-2-benzylidenessigsäure und 7-Amino-3-(3-carboxypyridinium)methyl-3-cephem-4-carbonsäurehydrochlorid-hydrat.

$^1$H-NMR ($D_2O$) δ (ppm) = 9,18 (1) s, 8,90 (1) d, J = 7 Hz, 8,77 (1) d, J = 7 Hz, 7,99 (1) t, J = 7 Hz, 7,31 (5) bs, 7,20 (1) s, 6,54 (1) s, 5,73 (1) d, J = 5 Hz, 5,49 (1) d, J = 14 Hz, 5,24 (1) d, J = 14 Hz, 5,12 (1) d, J = 5 Hz, 3,47 (1) d, J = 17 Hz, 3,00 (1) d, J = 17 Hz.

Beispiel 42

7-[Z-2-(2-Aminothiazol-4-yl)-2-benzylidenacetamido]-3-(4-hydroxymethylpyridinium)methyl-3-cephem-4-carboxylat

Die Herstellung erfolgte analog Beispiel 38 aus Z-2-(2-Aminothiazol-4-yl)-2-benzylidenessigsäure und 7-Amino-3-(4-hydroxymethylpyridinium)-3-cephem-4-carbonsäurehydrochlorid-hydrat.

$^1$H-NMR (DMSO/$CD_3OD$) δ (ppm) = 9,23 (2) d, J = 7 Hz, 8,00 (2) d, J = 7 Hz, 7,1–7,5 (6) m, 6,38 (1) s, 5,70 (1) d, J = 5 Hz, 5,63 (1) d, J = 13 Hz, 5,12 (1) d, J = 5 Hz, 5,10 (1) d, J = 13 Hz, 4,77 (2) bs, 3,50 (1) d, J = 18 Hz, 3,04 (1) d, J = 18 Hz.

Beispiel 43

7-Amino-3-[4-(3-hydroxypropyl)pyridinium]methyl-3-cephem-4-carboxylat

Die Herstellung erfolgte analog Beispiel 1.

$^1$H-NMR ($D_2O$) δ (ppm) = 8,82 (2H, d, J = 8 Hz, H-2,6-Py); 8,00 (2H, d, J = 8 Hz, H-3,5-Py); 5,54 (1H, d, J = 14 Hz, $CH_2$-Py); 5,37 (1H, d, J = 14 Hz, $CH_2$-Py); 5,35 (1H, d, J = 5 Hz, H-7-Lactam); 5,23 (1H, d, J = 5 Hz, H-6-Lactam); 3,72 (1H, d, J = 18 Hz, S-$CH_2$); 3,68 (2H, t, J = 7 Hz, Py-$CH_2$); 3,35 (1H, d, J = 18 Hz, S-$CH_2$); 3,05 (2H, t, J = 7 Hz, –$CH_2$–OH); 2,01 (2H, m,—$CH_2$–).

Beispiel 44

7-[1-(2-Aminothiazol-4-yl)-1(Z)-propencarboxamido]-3-[4-(3-hydroxypropyl)pyridinium]-methyl-3-cephem-4-carboxylat

Die Herstellung erfolgte analog Beispiel 15.

$^1$H-NMR ($D_6$-DMSO) δ (ppm) = 9,35 (2H, d, J = 8 Hz, H-2,6-Py); 9,18 (1H, d, J = 9 Hz, NH); 8,03 (2H, d, J = 8 Hz, H-3,5-Py); 6,97 (2H, bs, $NH_2$); 6,30 (1H, q, J = 8 Hz, C = C–H); 6,18 (1H, s, Thiazol); 5,69 (1H, dd, J = 9 Hz, J = 5 Hz, H-7-Lactam); 5,62 (1H, d, J = 14 Hz, $CH_2$-Py); 5,10 (1H, d, J = 5 Hz, H-6-Lactam); 5,01 (1H, d, J = 14 Hz, $CH_2$-Py); 3,52 (1H, d, J = 18 Hz, S-$CH_2$); 3,44 (2H, m, Py-$CH_2$-); 3,03 (1H, d, J = 18 Hz, S-$CH_2$); 2,90 (2H, m, –$CH_2$–OH); 1,80 (2H, m, –$CH_2$-); 1,75 (3H, d, J = 8 Hz, C = C–$CH_3$).

Beispiel 45

7-Amino-3-[3-(3-hydroxypropyl)pyridinium]methyl-3-cephem-4-carboxylat

Die Herstellung erfolgte analog Beispiel 1.

$^1$H-NMR ($D_2O$) δ (ppm) = 8,73 (1H, s, H-2-Py); 8,69 (1H, d, J = 7 Hz, H-6-Py); 8,37 (1H, d, J = 8 Hz, H-4-Py); 7,92 (1H, dd, J = 8 Hz, J = 7 Hz, H-5-Py); 5,53 (1H, d, J = 14 Hz, $CH_2$-Py); 5,24 (1H, d, J = 5 Hz, H-7-Lactam); 5,13 (1H, d, J = 5 Hz, H-6-Lactam); 5,08 (3H, m, Py-$CH_2$-; S-$CH_2$); 3,26 (1H, d, J = 18 Hz, S-$CH_2$); 2,85 (2H, t, J = 7 Hz, –$CH_2$–OH); 1,86 (2H, m, –$CH_2$-).

Beispiel 46

7-[1-(2-Aminothiazol-4-yl)-1(Z)-propencarboxymido]-3-[3-(3-hydroxypropyl)pyridinium]-methyl-3-cephem-4-carboxylat

Die Herstellung erfolgte analog Beispiel 15

$^1H$-NMR ($D_6$-DMSO) δ (ppm) = 9,35 (1H, d, J=7 Hz, H-6-Py); 9,23 (1H, s, H-2-Py); 9,11 (1H, d, J=9 Hz, NH); 8,40 (1H, d, J=8 Hz, H-4-Py); 8,02 (1H, dd, J=8 Hz, J=7 Hz, H-5-Py); 6,92 (2H, bs, $NH_2$); 6,24 (1H, q, J=8 Hz, C=C–H); 6,13 (1H, s, Thiazol); 5,60 (2H, m, H-7-Lactam, $CH_2$-Py); 5,05 (1H, d, J=5 Hz, H-6-Lactam); 5,02 (1H, d, J=14 Hz, $CH_2$-Py); 3,50 (1H, d, J=18 Hz, S-$CH_2$); 3,42 (2H, m, Py-$CH_2$); 3,05 (1H, d, J=18 Hz, S-$CH_2$); 2,80 (2H, m, $CH_2$–OH); 1,78 (2H, m, –$CH_2$-); 1,73 (3H, d, J=8 Hz, C=C–$CH_3$).

Beispiel 47
7-Amino-3-[4-(2-hydroxyethyl)pyridinium]methyl-3-cephem-4-carboxylat

Die Herstellung erfolgte analog Beispiel 1.

$^1H$-NMR ($D_2O$) δ (ppm) = 8,75 (2H, d, J=7 Hz, H-2,6-Py); 7,91 (2H, d, J=7 Hz, H-3,5-Py); 5,54 (1H, d, J=14 Hz, $CH_2$-Py); 5,27 (1H, d, J=14 Hz, $CH_2$-Py); 5,25 (1H, d, J=5 Hz, H-7-Lactam); 5,14 (1H, d, J=5 Hz, H-6-Lactam); 3,88 (2H, t, J=6 Hz, Py-$CH_2$); 3,62 (1H, d, J=18 Hz, S-$CH_2$); 3,27 (1H, d, J=18 Hz, S-$CH_2$); 3,08 (2H, t, J=6 Hz, $CH_2$–OH).

Beispiel 48
7-[1-(2-Aminothiazol-4-yl)-1(Z)-propencarboxamido]-3-[4-(2-hydroxyethyl)pyridinium]methyl-3-cephem-4-carboxylat

Die Herstellung erfolgte analog Beispiel 15.

$^1H$-NMR ($D_6$-DMSO) δ (ppm) = 9,35 (2H, d, J=6 Hz, H-2,6-Py); 9,21 (1H, d, J=9 Hz, NH); 8,05 (2H, d, J=6 Hz, H-3,5-Py); 7,00 (2H, bs, $NH_2$); 6,30 (1H, q, J=8 Hz, C=C–H); 6,18 (1H, s, Thiazol); 6,69 (1H, dd, J=9 Hz, J=5 Hz, H-7-Lactam); 6,62 (1H, d, J=13 Hz, $CH_2$-Py); 5,12 (1H, d, J=5 Hz, H-6-Lactam); 5,08 (1H, d, J=13 Hz, $CH_2$-Py); 3,77 (2H, z, J=6 Hz, Py-$CH_2$); 3,56 (1H, d, J=18 Hz, S-$CH_2$); 3,05 (1H, d, J=18 Hz, S-$CH_2$); 3,02 (2H, m, $CH_2$–OH); 1,75 (3H, d, J=8 Hz, C=C–$CH_3$).

Beispiel 49
7-[1-(2-Aminothiazol-4-yl)-1(Z)-propencarboxamido]-3-[2-(2-hydroxyethyl)pyridinium]methyl-3-cephem-4-carboxylat

Die Herstellung erfolgte analog Beispiel 15.

$^1H$-NMR ($D_6$-DMSO) δ (ppm) = 9,45 (1H, d, J=7 Hz, H-6-Py); 9,21 (1H, d, J=9 Hz, NH); 8,47 (1H, m, H-4-Py); 8,02 (2H, m, H-3,5-Py); 6,98 (2H, bs, $NH_2$); 6,29 (1H, q, J=8 Hz, C=C–H); 6,17 (1H, s, Thiazol); 5,67 (1H, dd, J=9 Hz, J=5 Hz, H-7-Lactam); 5,50 (2H, m, $CH_2$-Py); 5,07 (1H, d, J=5 Hz, H-6-Lactam); 3,81 (2H, t, Py-$CH_2$); 3,45 (1H, d, J=18 Hz, S-$CH_2$); 3,37 (2H, m, $CH_2$–OH); 3,05 (1H, d, J=18 Hz, S-$CH_2$); 1,74 (3H, d, J=8 Hz, C=C-$CH_3$).

Beispiel 50
7-Amino-3-[4-(1-hydroxyethyl)pyridinium]methyl-3-cephem-4-carboxylat

Die Herstellung erfolgte analog Beispiel 1.

$^1H$-NMR ($D_2O$) δ (ppm) = 8,90 (2H, d, J=7 Hz, H-2,6-Py); 8,01 (2H, d, J=7 Hz, H-3,5-Py); 5,57 (1H, d, J=15 Hz, $CH_2$-Py); 5,38 (1H, d, J=15 Hz, $CH_2$-Py); 5,35 (1H, d, J=5 Hz, H-7-Lactam); 5,22 (2H, m, CH–$CH_3$, H-6-Lactam); 3,72 (1H, d, J=18 Hz, S-$CH_2$); 3,35 (1H, d, J=18 Hz, S-$CH_2$); 1,52 (3H, d, J=7 Hz, CH–$CH_3$).

Beispiel 51
7-[1-(2-Aminothiazol-4-yl)-1(Z)-propencarboxamido]-3-[4-(1-hydroxyethyl)pyridinium]methyl-3-cephem-4-carboxylat

Die Herstellung erfolgte analog Beispiel 15.

$^1H$-NMR ($D_6$-DMSO) δ (ppm) = 9,41 (2H, d, J=7 Hz, H-2,6-Py); 9,21 (1H, d, J=9 Hz, NH); 8,12 (2H, d, J=7 Hz, H-3,5-Py); 7,01 (2H, bs, $NH_2$); 6,32 (1H, q, J=8 Hz, C=C–H); 6,21 (1H s, Thiazol); 5,68 (2H, m, H-7-Lactam, $CH_2$-Py); 5,13 (1H, d, J=5 Hz, H-6-Lactam); 5,04 (2H, m, $CH_2$-Py, CH–$CH_3$); 3,56 (1H, d, J=18 Hz, S-$CH_2$); 3,08 (1H, d, J=18 Hz, S-$CH_2$); 1,77 (3H, d, J=8 Hz, C=C–$CH_3$); 1,42 (3H, d, J=7 Hz, CH–$CH_3$).

Beispiel 52
7-[1-(2-Aminothiazol-4-yl)-1(Z)-propencarboxamido]-3-[2-(2-propyl-1,3-diol)pyridinium]methyl-3-cephem-4-carboxylat

Die Herstellung erfolgte analog Beispiel 15.

$^1H$-NMR ($D_6$-DMSO) δ (ppm) = 9,54 (1H, d, J=7 Hz, H-6-Py); 9,25 (1H, d, J=9 Hz, NH); 8,55 (1H, m, H-4-Py); 8,19 (1H, d, J=7 Hz, H-3-Py); 8,06 (1H, m, H-5-Py); 7,02 (2H, bs, $NH_2$); 6,32 (1H, q, J=8 Hz, C=C–H); 6,21 (1H, s, Thiazol); 5,70 (2H, m, H-7-Lactam, $CH_2$-Py); 5,52 (1H, d, J=14 Hz, $CH_2$-Py); 5,11 (1H, d, J=5 Hz, H-6-Lactam); 4,02 (1H, m, Py-CH); 3,84 (2H, m, $CH_2$–OH); 3,72 (2H, m $CH_2$–OH); 3,45 (1H, d, J=18 Hz, S-$CH_2$); 3,02 (1H, d, J=18 Hz, S-$CH_2$); 1,77 (3H, d, J=8 Hz, C=C–$CH_3$).

Beispiel 53
7-Amino-3-(6-hydroxymethylchinolinium)methyl-3-cephem-4-carboxylat

Die Herstellung erfolgte analog Beispiel 1.

$^1H$-NMR ($D_2O$) δ (ppm) = 9,23 (1H, d, J=7 Hz, H-2-Chin.); 9,16 (1H, d, J=8 Hz, Chin.); 8,42 (1H, d, J=8 Hz, Chin.); 8,31 (1H, s, Chin.); 8,18 (1H, d, J=8 Hz, Chin.); 8,06 (1H, dd, J=7 Hz, J=8 Hz, Chin.); 6,03 (1H, d, J=15 Hz, $CH_2$-Chin.); 5,89 (1H, d, J=15 Hz, $CH_2$-Chin.); 5,24 (1H, d, J=5 Hz, H-7-Lactam); 5,17, (1H, d, J=5 Hz, H-6-Lactam); 4,91 (2H, s, $CH_2$–OH); 3,45 (1H, d, J=18 Hz, S-$CH_2$); 3,27 (1H, d, J=18 Hz, S-$CH_2$).

Beispiel 54
7-[1-(2-Aminothiazol-4-yl)-1(Z)-propencarboxamido]-3-(6-hydroxymethylchinolinium)-methyl-3-cephem-4-carboxylat

$^1H$-NMR ($D_6$-DMSO) δ (ppm) = 9,73 (1H, d, J=7 Hz, H-2-Chin.); 9,34 (1H, d, J=8 Hz, Chin.); 9,26 (1H, d, J=8 Hz, Chin.); 9,13 (1H, d, J=9 Hz, NH); 8,42 (1H, s, Chin.); 8,25 (1H, dd, J=7 Hz, J=8 Hz, Chin.); 8,18 (1H, d, J=8 Hz, Chin.); 7,03 (2H, bs, $NH_2$); 6,34 (1H, q, J=8 Hz, C=CH); 6,22 (1H, s, Thiazol); 6,08 (1H, d, J=15 Hz, $CH_2$-Chin.); 5,96 (1H, d, J=15 Hz, $CH_2$-Chin.); 5,71 (1H, dd, J=9 Hz, J=5 Hz, H-7-Lactam); 5,11 (1H, d, J=5 Hz, H-6-Lactam); 4,86 (2H, s, –$CH_2$OH); 3,42 (1H, d, J=18 Hz, S-$CH_2$); 3,01 (1H, d, J=18 Hz, S-$CH_2$); 1,78 (3H, d, J=8 Hz, C=C–$CH_3$).

Die Herstellung erfolgte analog Beispiel 15.

Beispiel 55

7-Amino-3-(3-formamidopyridinium)methyl-3-cephem-4-carboxylat

$^1$H-NMR ($D_2O$) δ (ppm) = 9,53 (1H, s, CHO); 8,70 (1H, d, J = 7 Hz, H-6-Py); 8,43 (2H, m, H-2,4-Py); 8,01 (1H, dd, J = 8 Hz, J = 7 Hz, H-5-Py); 5,65 (1H, d, J = 14 Hz, $CH_2$-Py); 5,35 (1H, d, J = 14 Hz, $CH_2$-Py); 5,30 (1H, d, J = 5 Hz, H-7-Lactam); 5,19 (1H, d, J = 5 Hz, H-6-Lactam); 3,71 (1H, d, J = 18 Hz, S-$CH_2$); 3,34 (1H, d, J = 18 Hz, S-$CH_2$).

Beispiel 56

7-[1-(2-Aminothiazol-4-yl)-1(Z)-propencarboxamido]-3-(3-formamidopyridinium)methyl-3-cephem-4-carboxylat

Die Herstellung erfolgte analog Beispiel 15.

$^1$H-NMR ($D_6$-DMSO) δ (ppm) = 9,68 (1H, s, CHO); 9,25 (2H, m, NH, H-6-Py); 8,78 (1H, d, J = 8 Hz, H-4-Py); 8,56 (1H, s, H-2-Py); 8,13 (1H, m, H-5-Py); 7,01 (2H, bs, $NH_2$); 6,32 (1H, q, J = 8 Hz, C = C–N); 6,20 (1H, s, Thiazol); 5,72 (2H, m, $CH_2$-Py, H-7-Lactam); 5,25 (1H, d, J = 14 Hz, $CH_2$-Py); 5,13 (1H, d, J = 5 Hz, H-6-Lactam); 3,57 (1H, d, J = 18 Hz, S-$CH_2$); 3,16 (1H, d, J = 18 Hz, S-$CH_2$); 1,76 (3H, d, J = 8 Hz, C = C–$CH_3$).

Beispiel 57

7-[1-(2-Aminothiazol-4-yl)-1(Z)-propencarboxamido]-3-(3-aminopyridinium)methyl-3-cephem-4-carboxylat

Eine Lösung von 500 mg 7-[1-(2-Aminothiazol-4-yl)-1(Z)-propencarboxamido]-3-(3-formylamidopyridinium)methyl-3-cephem-4-carboxylat in 6 ml Methanol und 0,3 ml konz. Salzsäure wird 5 h bei Raumtemperatur gerührt. Das Lösungsmittel wird im Vakuum abgezogen, der Rückstand in wenig Wasser aufgenommen, mit Ionenaustauscher MP 62 neutralisiert und über Adsorberharz HP 20 gereinigt (Elution mit Waser und steigenden Mengen Acetonitril).

Ausbeute: 240 mg.

$^1$H-NMR ($D_6$-DMSO) δ (ppm) = 9,20 (1H, d, J = 9 Hz, NH); 8,58 (1H, s, H-2-Py); 8,46 (1H, d, J = 7 Hz, H-6-Py); 7,71 (1H, dd, J = 7 Hz, H = 8 Hz, H-5-Py); 7,57 (1H, d, J = 8 Hz, H-4-Py); 7,01 (2H, bs, $NH_2$); 6,73 (2H, bs, $NH_2$); 6,31 (1H, q, J = 8 Hz, C = C–H); 6,19 (1H, s, Thiazol); 5,67 (1H, dd, J = 9 Hz, J = 5 Hz, H-7-Lactam); 5,59 (1H, d, J = 14 Hz, $CH_2$-Py); 5,08 (1H, d, J = 5 Hz, H-6-Lactam); 4,95 (1H, d, J = 14 Hz, $CH_2$-Py); 4,48 (1H, d, J = 18 Hz, S-$CH_2$); 3,00 (1H, d, J = 18 Hz, S-$CH_2$); 1,76 (3H, d, J = 8 Hz, C = C–$CH_3$).

**Patentansprüche**

1. Verbindungen der allgemeinen Formel I

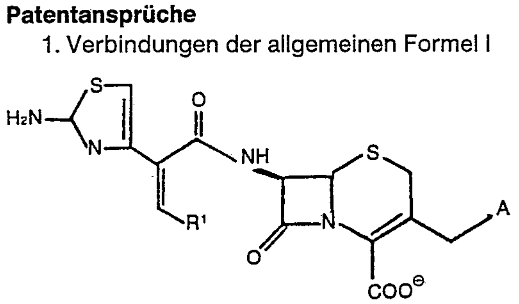

(I)

in der R[1] $C_1$–$C_6$-Alkyl, Phenyl oder Halogen-substituiertes Phenyl bedeutet und

A einen Pyridiniumrest

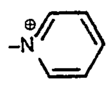

bedeutet, der 1- bis 3fach, gleich oder verschieden substituiert ist,

durch $C_1$–$C_6$-Alkyl, das ein- oder mehrfach substituiert ist durch Hydroxy, Carboxy, $C_1$–$C_6$-Alkyloxycarbonyl, Formyl oder $C_1$–$C_6$-Alkylcarbonyl, deren Carbonylgruppe auch in ketalisierter Form vorliegen kann, Carbamoyl, N-Hydroxy-carbamoyl, Sulfo, $C_1$–$C_6$-Alkyloxy, Hydroxy-$C_1$–$C_6$-alkyloxy, $C_1$–$C_6$-Alkylthio, $C_1$–$C_6$-Alkylsulfinyl, $C_1$–$C_6$-Alkylsulfonyl, $C_2$–$C_6$-Alkenyloxy, $C_2$–$C_6$-Alkenylthio, $C_2$–$C_6$-Alkenylsulfinyl oder $C_2$–$C_6$-Alkenylsulfonyl,

durch Cyano-$C_1$–$C_3$-alkyl, Epoxy-$C_2$–$C_6$-alkyl, Trifluormethyl oder Pentafluorethyl,

durch Hydroximino-methyl oder $C_1$–$C_4$-Alkoximinomethyl,

durch $C_2$–$C_6$-Alkenyl, das durch Hydroxy substituiert sein kann,

durch $C_2$–$C_6$-Alkinyl,

durch $C_3$–$C_7$-Cycloalkyl oder $C_3$–$C_7$-Cycloalkylmethyl, wobei in beiden Substituenten der Ring auch substituiert sein kann durch Hydroxy, Halogen, Carboxy, $C_1$–$C_6$-Alkyloxycarbonyl oder Cyano,

durch $C_4$–$C_7$-Cycloalkenyl,

durch $C_1$–$C_6$-Alkoxy, das durch Hydroxy, Carboxy oder $C_1$–$C_6$-Alkoxycarbonyl substituiert sein kann,

durch Epoxy-$C_2$–$C_6$-Alkoxy,

durch $C_2$–$C_6$-Alkenyloxy oder $C_2$–$C_6$-Alkinyloxy,

durch Phenoxy,

durch Amino, das gegebenenfalls ein- oder zweimal substituiert sein kann durch $C_1$–$C_6$-Alkyl, Formyl, $C_1$–$C_6$-Alkylcarbonyl, $C_1$–$C_6$-Alkoxycarbonyl, Carbamoyl, $C_1$–$C_6$-Alkylsulfonyl,

durch Cyano, Hydroxy oder Mercapto,

durch $C_1$–$C_6$-Alkylthio, $C_1$–$C_6$-Alkylsulfinyl oder $C_1$–$C_6$-Alkylsulfonyl, die im Alkylteil durch Hydroxy substituiert sein können,

durch Methylthio, Methylsulfinyl oder Methylsulfonyl, die im Methylteil substituiert sind durch Carboxy oder $C_1$–$C_6$-Alkyloxycarbonyl,

durch $C_2$–$C_6$-Alkenylthio, $C_2$–$C_6$-Alkenylsulfinyl oder $C_2$–$C_6$-Alkenylsulfonyl,

durch Phenyl, Benzyl, die auch durch Halogen substituiert sein können,

durch Sulfamoyl, das am Stickstoff einmal substituiert sein kann, durch $C_1$–$C_6$-Alkylaminocarbonyl, an den ein oder zwei gegebenenfalls substituierte 3- bis 7gliedrige Ringe ankondensiert sein können, die jeweils bis zu zwei Heteroatome aus der Gruppe O, N und S und bis zu zwei Doppelbindungen enthalten können und auch aromatisch oder heteroaromatisch sein können, wobei folgende Substituenten in Betracht kommen:

$C_1$–$C_6$-Alkyl, $C_3$–$C_7$-Cycloalkyl, $C_1$–$C_4$-Alkoxy, $C_1$–$C_4$-Hydroxyalkyl, Halogen, Hydroxy, Oxo, Hydroximino, Exomethylen, Carboxy, $C_1$–$C_6$-Alkyloxycarbonyl, Cyano, Carbamoyl, Sulfamoyl, Amino, $C_1$–$C_4$-Alkylamino oder $C_1$–$C_4$-Dialkylamino; deren

pharmazeutisch verträgliche Salze und deren unter physiologischen Bedingungen spaltbare Ester.

2. Verfahren zur Herstellung von Verbindungen der allgemeinen Formel I

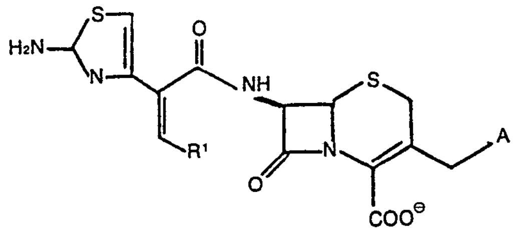

(I)

in der $R^1$ und A die in Anspruch 1 angegebene Bedeutung haben, dadurch gekennzeichnet, daß man eine Verbindung der Formel II

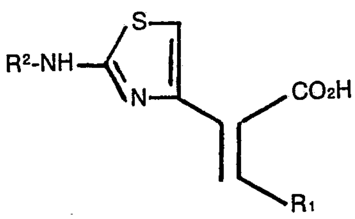

(II)

in der
$R^1$ die in Anspruch 1 genannte Bedeutung hat und
$R^2$ eine Aminschutzgruppe darstellt,
zunächst mit einer Verbindung der Formel VII

$$X{-}SO_2R^3 \qquad (VII)$$

umsetzt, in der bedeuten:
X Cl, Br oder $OSO_2R^3$ und
$R^3$ einen Alkylrest mit 1 bis 10 C-Atomen, der gegebenenfalls durch Fluor, Chlor, CN, Phenyl, Alkyloxycarbonyl, Alkyloxy oder Alkyl substituiert sein kann, wobei letztere Alkylreste 1 bis 4 C-Atome tragen können, oder ein Phenylrest, der gegebenenfalls durch Fluor, Chlor, Brom, CN, Alkyl, Alkyloxy, Alkylthio, Alkyloxycarbonyl – wobei letztere Alkylgruppen 1 bis 4 C-Atome tragen können – Nitro, Trifluormethyl und Phenyl substituiert sein kann, und die so erhaltenen Verbindungen der Formel III

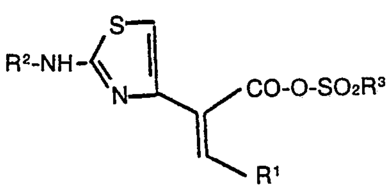

(III)

In der
$R^1$, $R^2$ und $R^3$ obengenannte Bedeutungen haben, mit einer Verbindung der Formel IV

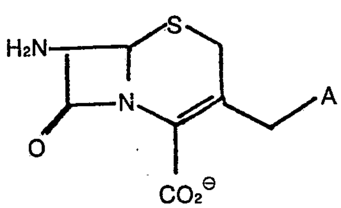

(IV)

umsetzt, in der
A die in Anspruch 1 angegebene Bedeutung hat, und aus den so erhaltenen Verbindungen der Formel V

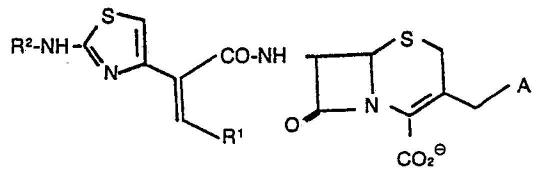

(V)

die Schutzgruppe $R^2$ abspaltet und gegebenenfalls in die Salze oder Ester überführt.

3. Verfahren zur Herstellung von Verbindungen der allgemeinen Formel I

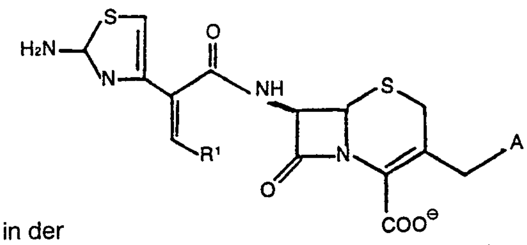

(I)

in der
$R^1$ und A die in Anspruch 1 angegebene Bedeutung haben, dadurch gekennzeichnet, daß man Verbindungen der Formel IX

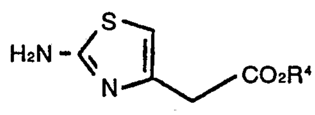

(IX)

in der
$R^4$ einen gegebenenfalls substituierten Alkyl-, Cycloalkyl-, Alkenyl-, Cycloalkenyl-, Aryl- oder Heterocyclylrest darstellt,
mit einer Verbindung der Formel

$$(R^5{-}O{-}CO)_2O$$

umsetzt, in der
$R^5$ die obengenannte Bedeutung von $R^4$ hat, anschließend die so erhaltene Verbindung der Formel X

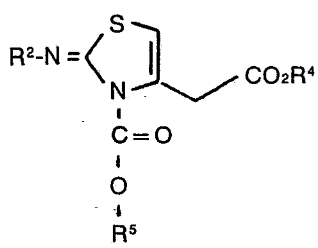

(X)

in der
$R^2$ den Rest $R^5{-}O{-}CO$ darstellt und $R^4$ und $R^5$ obengenannte Bedeutungen haben,
zunächst mit einer Base und dann mit einem Aldehyd der Formel

$$R^1{-}CHO$$

in der $R^1$ die obengenannte Bedeutung hat, zu einer Verbindung der Formel XI

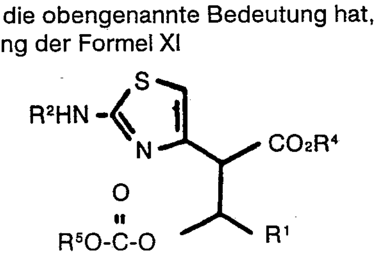

(XI)

umsetzt, in der $R^1$, $R^2$, $R^4$ und $R^5$ obengenannte Bedeutungen haben,
die Verbindung XI dann mit einer Base zur Verbindung XII umsetzt

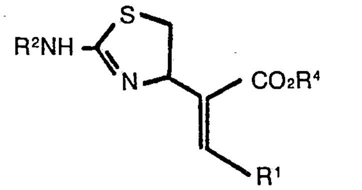
(XII)

in der
$R^1$, $R^2$ und $R^4$ obengenannte Bedeutungen haben, anschließend verseift zu einem Gemisch der Verbindungen der Formel II und IIa

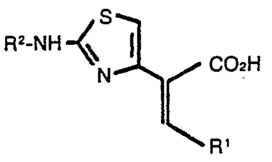
+
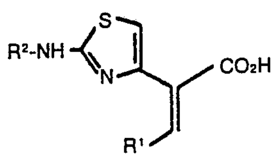

(II) (IIa)

oder selektiv verseift zu Verbindungen der Formel II, worin jeweils $R^1$ und $R^2$ die obengenannten Bedeutungen haben,
die Verbindungen der Formel II in Verbindungen der Formel III überführt

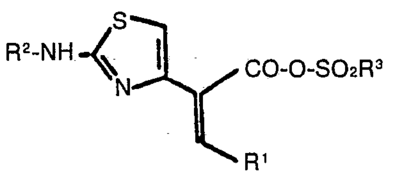
(III)

in der
$R^3$ einen Alkylrest mit 1 bis 10 C-Atomen, der gegebenenfalls durch Fluor, Chlor, CN, Phenyl, Alkyloxycarbonyl, Alkyloxy oder Alkyl substituiert sein kann, wobei letztere Alkylreste 1 bis 4 C-Atome tragen können, oder ein Phenylrest, der gegebenenfalls durch Fluor, Chlor, Brom, CN, Alkyl, Alkyloxy, Alkylthio, Alkyloxycarbonyl – wobei letztere Alkylgruppe 1 bis 4 C-Atome tragen können – Nitro, Trifluormethyl und Phenyl substituiert sein kann,
bedeutet, diese dann gemäß Anspruch 2 mit einer Verbindung der Formel IV in eine Verbindung der Formel V überführt und aus dieser die Schutzgruppe $R^2$ abspaltet und gegebenenfalls in die Salze oder Ester überführt.

4. Verfahren zur Herstellung von Verbindungen der allgemeinen Formel I

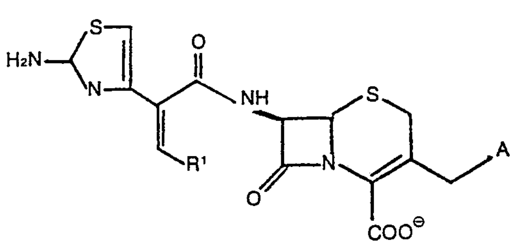
(I)

in der
$R^1$ und A die in Anspruch 1 angegebene Bedeutung haben, dadurch gekennzeichnet, daß man Verbindungen der Formel XV

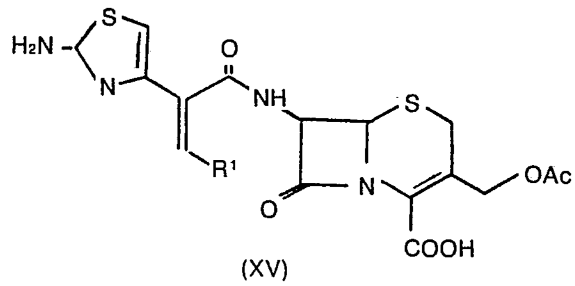
(XV)

mit einer Verbindung der Formel A', in der
A', die in Anspruch 1 angegebene Bedeutung von A hat, jedoch nicht die positive Ladung am Stickstoff trägt,
umsetzt.

5. Arzneimittel, enthalten mindestens eine Verbindung nach einem der Ansprüche 1 bis 4.

6. Verbindungen gemäß Anspruch 1 zur Anwendung in einem Verfahren zur therapeutischen Behandlung des menschlichen oder tierischen Körpers.

7. Verwendung der Verbindungen gemäß Anspruch 1 zur Herstellung von Arzneimitteln.

**Claims**

1. Compounds of the general formula I

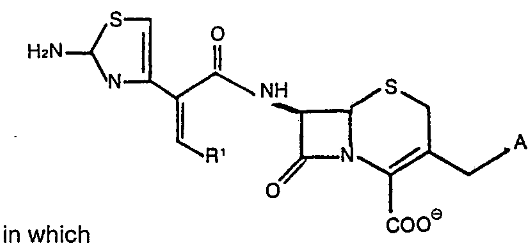
(I)

in which
$R^1$ denotes $C_1$–$C_6$-alkyl, phenyl or halogen substituted phenyl and
A denotes a pyridinium radical

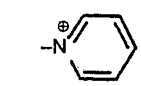

which is
mono-, di- or tri-substituted, by identical or different substituents as follows: by $C_1$–$C_6$-alkyl, which is monosubstituted or polysubstituted by hydroxyl, carboxyl, $C_1$–$C_6$-alkoxycarbonyl, formyl or $C_1$–$C_6$-alkylcarbonyl, the carbonyl group of which can also be in ketalised form, carbamoyl, N-hydroxycarbamoyl, sulpho, $C_1$–$C_6$-alkoxy, hydroxy-$C_1$–$C_6$-alkoxy, $C_1$–$C_6$-alkylthio, $C_1$–$C_6$-alkylsulphinyl, $C_1$–$C_6$-alkylsulphonyl, $C_2$–$C_6$-alkenyloxy, $C_2$–$C_6$-alkenylthio, $C_2$–$C_6$-alkenylsulphinyl or $C_2$–$C_6$-alkenylsulphonyl, by cyano-$C_1$–$C_3$-alkyl, epoxy-$C_2$–$C_6$-alkyl, trifluoromethyl or pentafluoroethyl, by hydroximinomethyl or $C_1$–$C_4$-alkoximinomethyl, by $C_2$–$C_6$-alkenyl, which can be substituted by hydroxyl, by $C_2$–$C_6$-alkinyl, by $C_3$–$C_7$-cycloalkyl or $C_3$–$C_7$-cycloalkylmethyl, it being possible for the ring in both substituents also to be substituted by hydroxyl, halogen, carboxyl, $C_1$–$C_6$-alkoxycarbonyl or cyano, by $C_4$–$C_7$-cycloalkenyl, by $C_1$–$C_6$-alkoxy, which can be substituted by hydroxyl, carboxyl or $C_1$–$C_6$-alkoxycarbonyl, by epoxy-$C_2$–$C_6$-alkoxy, by $C_2$–$C_6$-alkenyloxy or $C_2$–$C_6$-alkinyloxy, by

phenoxy, by amino, which can optionally be mono- or di-substituted by $C_1$–$C_6$-alkyl, formyl, $C_1$–$C_6$-alkylcarbonyl, $C_1$–$C_6$-alkoxycarbonyl, carbamoyl or $C_1$–$C_6$-alkylsulphonyl, by cyano, hydroxyl or mercapto, by $C_1$–$C_6$-alkylthio, $C_1$–$C_6$-alkylsulphinyl or $C_1$–$C_6$-alkylsulphonyl, each of which can be substituted in the alkyl part by hydroxyl, by methylthio, methylsulphinyl or methylsulphonyl, each of which is substituted in the methyl part by carboxyl or $C_1$–$C_6$-alkoxycarbonyl, by $C_2$–$C_6$-alkenylthio, $C_2$–$C_6$-alkenylsulphinyl or $C_2$–$C_6$-alkenylsulphonyl, by phenyl or benzyl, each of which can also be substituted by halogen, and by sulphamoyl, which can be monosubstituted on the nitrogen by $C_1$–$C_6$-alkylaminocarbonyl, and onto which one or two optionally substituted 3-membered to 7-membered rings can be fused, which can contain in each case up to two hetero-atoms from the group comprising O, N and S and up to two double bonds and can also be aromatic or heteroaromatic, possible substituents being the following: $C_1$–$C_6$-alkyl, $C_3$–$C_7$-cycloalkyl, $C_1$–$C_4$-alkoxy, $C_1$–$C_4$-hydroxyalkyl, halogen, hydroxyl, oxo, hydroximino, exomethylene, carboxyl, $C_1$–$C_6$-alkoxycarbonyl, cyano, carbamoyl, sulphamoyl, amino, $C_1$–$C_4$-alkylamino and $C_1$–$C_4$-dialkylamino, pharmaceutically acceptable salts thereof and esters thereof which can be split under physiological conditions.

2. Process for the preparation of compounds of the general formula I

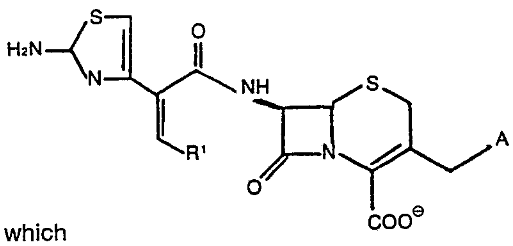

(I)

in which

$R^1$ and A have the meaning given in Claim 1, characterised in that a compound of the formula II

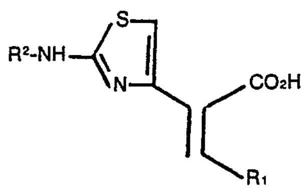

(II)

in which

$R^1$ has the meaning given in Claim 1 and

$R^2$ represents an amine-protective group,

is first reacted with a compound of the formula VII

$$X{-}SO_2R_3 \qquad (VII)$$

in which

X denotes Cl, Br or $OSO_2R^3$

$R^3$ denotes an alkyl radical which has 1–10 C atoms and can optionally be substituted by fluorine, chlorine, CN, phenyl, alkoxycarbonyl, alkoxy or alkyl, it being possible for the latter alkyl radicals to carry 1–4 C atoms, or denotes a phenyl radical, which can optionally be substituted by fluorine, chlorine, bromine, CN, alkyl, alkoxy, alkylthio, alkoxycarbonyl – it being possible for the latter alkyl groups to carry 1–4 C atoms – nitro, trifluoromethyl and phenyl,

and the compounds thus obtained, of the formula III

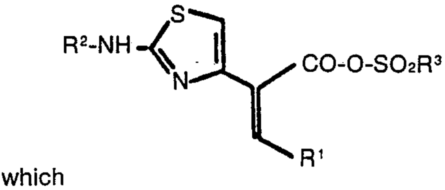

(III)

in which

$R^1$, $R^2$ and $R^3$ have the abovementioned meanings, are reacted with a compound of the formula IV

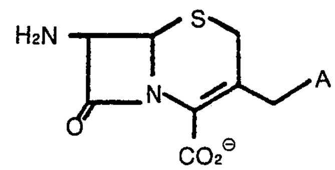

(IV)

in which

A has the meaning given in Claim 1,

and the protective group $R^2$ is split off from the compounds thus obtained, of the formula V

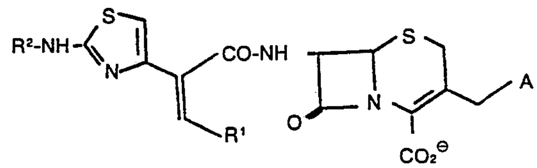

(V)

and, if appropriate, the products are converted into the salts or esters.

3. Process for the preparation of compounds of the general formula I

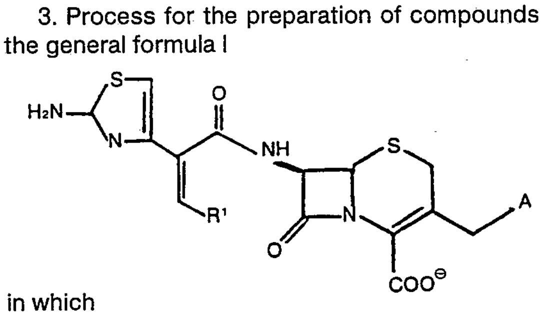

(I)

in which

$R^1$ and A have the meaning given in Claim 1,

characterised in that compounds of the formula IX

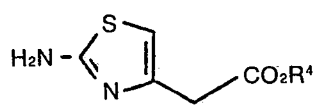

(IX)

in which

$R^4$ represents an optionally substituted alkyl, cycloalkyl, alkenyl, cycloalkenyl, aryl or heterocyclyl radical,

are reacted with a compound of the formula

$$(R^5{-}O{-}CO)_2O$$

in which

$R^5$ has the abovementioned meaning of $R^4$, the compound thus obtained, of the formula X

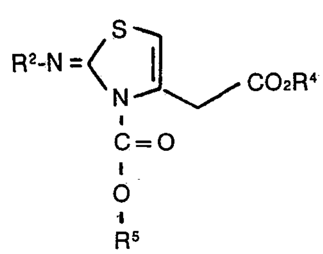

(X)

in which
$R^2$ represents the radical $R^5$–O–CO and
$R^4$ and $R^5$ have the abovementioned meanings,
is then reacted first with a base and then with an aldehyde of the formula

$R^1$–CHO

in which
$R^1$ has the abovementioned meaning,
to give a compound of the formula XI

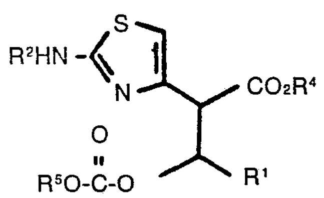

(XI)

in which
$R^1$, $R^2$, $R^4$ and $R^5$ have the abovementioned meanings,
the compound XI is then reacted with a base to give the compound XII

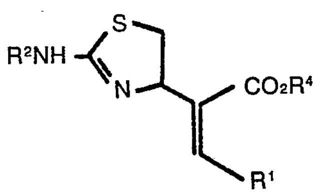

(XII)

in which
$R^1$, $R^2$ and $R^4$ have the abovementioned meanings, which is then hydrolysed to give a mixture of the compounds of the formula II and IIa

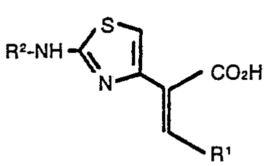

+

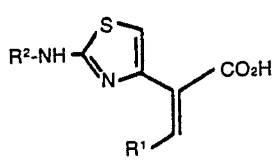

(II) (IIa)

or is hydrolysed selectively to give compounds of the formula II,
wherein, in each case,
$R^1$ and $R^2$ have the abovementioned meanings,
the compounds of the formula II are converted into compounds of the formula III

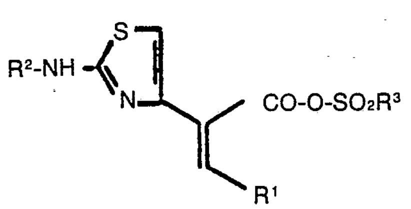

(III)

in which
$R^3$ denotes an alkyl radical which has 1–10 C atoms and can optionally be substituted by fluorine, chlorine, CN, phenyl, alkoxycarbonyl, alkoxy or alkyl, it being possible for the latter alkyl radicals to carry 1–4 C atoms, or denotes a phenyl radical, which can optionally be substituted by fluorine, chlorine, bromine, CN, alkyl, alkoxy, alkylthio, alkoxycarbonyl – it being possible for the latter alkyl group to carry 1–4 C atoms – nitro, trifluoromethyl and phenyl,
these are then converted into a compound of the formula V with a compound of the formula IV in accordance with Claim 2, and the protective group $R^2$ is split off from the compound of the formula V and, if appropriate, the product is converted into the salts or esters.

4. Process for the preparation of compounds of the general formula I

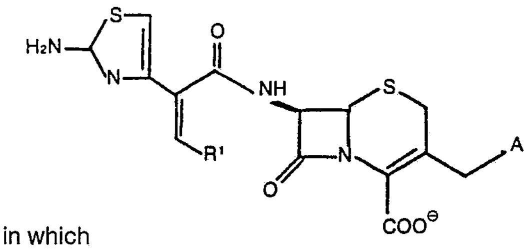

(I)

in which
$R^1$ and A have the meaning given in Claim 1,
characterised in that compounds of the formula XV

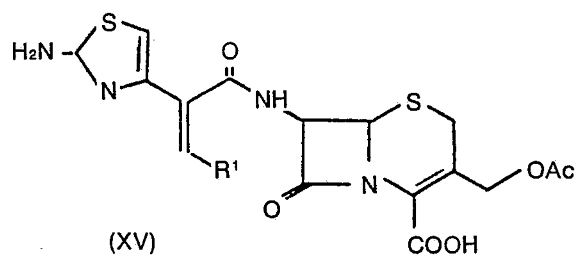

(XV)

are reacted with a compound of the formula A'
in which
A' has the meaning of A given in Claim 1, but does not carry the positive charge on the nitrogen.

5. Medicaments containing at least one compound according to one of Claims 1–4.

6. Compounds according to Claim 1 for use in a process for the therapeutic treatment of the human or animal body.

7. Use of the compounds according to Claim 1 for the preparation of medicaments.

**Revendications**

1. Composés de formule générale I

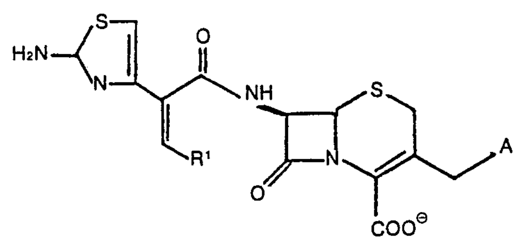

(I)

dans laquelle
$R^1$ représente un groupe alkyle en $C_1$–$C_6$, phényle ou phényle substitué par de l'halogène et,
A représente un reste pyridinium

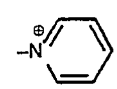

qui peut porter 1 à 3 substituants, identiques ou différents, qui sont:
un groupe alkyle en $C_1$–$C_6$, qui peut être substitué une ou plusieurs fois par un groupe hydroxy, carboxy, alkyloxy(en $C_1$–$C_6$)carbonyle, formyle ou alkyl-

(en $C_1$–$C_6$)carbonyle, dont le groupe carbonyle peut être également présent sous forme cétalisée, carbamoyle, N-hydroxycarbamoyle, sulfo, alkyloxy en $C_1$–$C_6$, hydroxy-alkyloxy en $C_1$ à $C_6$, alkylthio en $C_1$–$C_6$, alkylsulfinyle en $C_1$–$C_6$, alkylsulfonyle en $C_1$–$C_6$, alcényloxy en $C_2$–$C_6$, alcénylthio en $C_2$–$C_6$, alcénylsulfinyle en $C_2$–$C_6$ ou alcénylsulfonyle en $C_2$–$C_6$,
un groupe cyano-alkyle en $C_1$–$C_3$, époxy-alkyle en $C_2$–$C_6$, trifluorométhyle ou pentafluoréthyle,
un groupe hydroxy-iminométhyle ou alcoxy(en $C_1$–$C_4$)iminométhyle,
un groupe alcényle en $C_2$–$C_6$, qui peut être substitué par un groupe hydroxyle,
un groupe alcynyle en $C_2$–$C_6$,
un groupe cycloalkyle en $C_3$–$C_7$ ou cycloalkyl(en $C_3$–$C_7$)méthyle,
le noyau de ces deux groupes substituants pouvant également être substitué par un groupe hydroxy, halogéno, carboxy, alkyloxy(en $C_1$–$C_6$)carbonyle ou cyano,
un groupe cycloalcényle en $C_4$–$C_7$,
un groupe alcoxy en $C_1$–$C_6$, qui peut être substitué par un groupe hydroxyle, carboxy ou alcoxy(en $C_1$–$C_6$)carbonyle,
un groupe époxy-alcoxy en $C_2$–$C_6$,
un groupe alcényloxy en $C_2$–$C_6$ ou alcynyloxy en $C_2$–$C_6$,
un groupe phénoxy,
un groupe amino, qui peut être substitué éventuellement une ou deux fois par un groupe alkyle en $C_1$–$C_6$, formyle, alkyl(en $C_1$–$C_6$)carbonyle, alcoxy(en $C_1$–$C_6$)carbonyle, carbamoyle, alkylsulfonyle en $C_1$–$C_6$,
un groupe cyano, hydroxy ou mercapto,
un groupe alkylthio en $C_1$–$C_6$, alkylsulfinyle en $C_1$–$C_6$ ou alkylsulfonyle en $C_1$–$C_6$, qui peuvent être substitués dans la partie alkyle par des groupes hydroxy,
un groupe méthylthio, méthylsulfinyle ou méthylsulfonyle, qui sont substitués, dans la partie méthyle, par un groupe carboxy ou par un groupe alkyloxy(en $C_1$–$C_6$)carbonyle,
un groupe alcénylthio en $C_2$–$C_6$, alcénylsulfinyle en $C_2$–$C_6$ ou alcénylsulfonyle en $C_2$–$C_6$,
un groupe phényle ou benzyle, qui peuvent également être substitués par de l'halogène,
un groupe sulfamoyle, qui peut être substitué sur l'azote une fois par un groupe alkyl(en $C_1$–$C_6$)aminocarbonyle,
sur lesquels un ou deux noyaux à 3 à 7 chaînons, éventuellement substitués, peuvent être condensés et qui peuvent chaque fois contenir jusqu'à deux hétéroatomes choisis parmi O, N et S et jusqu'à deux doubles liaisons, et qui peuvent également être aromatiques ou hétéroaromatiques, les substituants suivants étant alors pris en compte: un groupe alkyle en $C_1$–$C_6$, cycloalkyle en $C_3$–$C_7$, alcoxy en $C_1$–$C_4$, hydroxyalkyle en $C_1$–$C_4$, halogéno, hydroxy, oxo, hydroxy-imino, exométhylène, carboxy, alkyloxy(en $C_1$–$C_6$)carbonyle, cyano, carbamoyle, sulfamoyle, amino, alkylamino en $C_1$–$C_4$ ou dialkyl(en $C_1$–$C_4$)-amino, leurs sels pharmaceutiquement tolérables et leurs esters scindables dans des conditions physiologiques.

2. Procédé pour préparer des composés de formule générale I

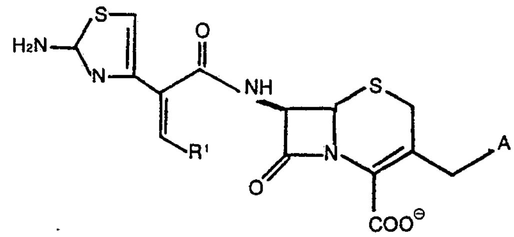

(I)

dans laquelle $R^1$ et A ont le sens indiqué à la revendication 1,
procédé caractérisé en ce qu'on fait réagir un composé de formule II

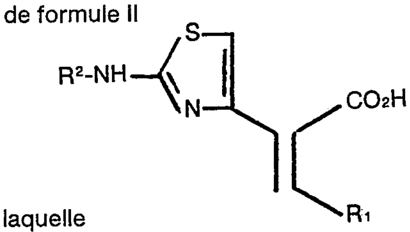

(II)

dans laquelle
$R^1$ a le sens indiqué à la revendication 1, et
$R^2$ représente un groupe protecteur de fonction amine,
tout d'abord avec un composé de formule VII

$$X\text{–}SO_2R^3 \qquad (VII)$$

dans laquelle
X représente Cl, Br ou $OSO_2R^3$, et
$R^3$ représente un reste alkyle ayant 1 à 10 atomes de carbone, qui peut éventuellement être substitué par du fluor, du chlore, un groupe CN, phényle, alkyloxycarbonyle, alkyloxy ou alkyle, ces derniers restes alkyle pouvant porter 1 à 4 atomes de carbone, ou un reste phényle, qui peut éventuellement être substitué par du fluor, du chlore, du brome, un groupe CN, alkyle, alkyloxy, alkylthio, alkyloxycarbonyle (ces derniers groupes alkyle pouvant comporter 1 à 4 atomes de carbone), nitro, trifluorométhyle et phényle, et l'on fait réagir les composés ainsi obtenus, de formule III

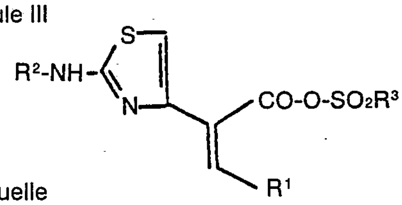

(III)

dans laquelle
$R^1$, $R^2$ et $R^3$ ont les sens précités,
avec un composé de formule IV

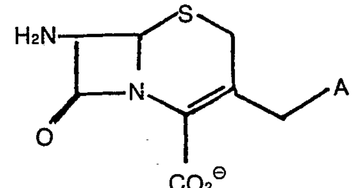

(IV)

dans laquelle
A a le sens indiqué à la revendication 1, et
on scinde le groupe protecteur $R^2$ des composés ainsi obtenus, de formule V

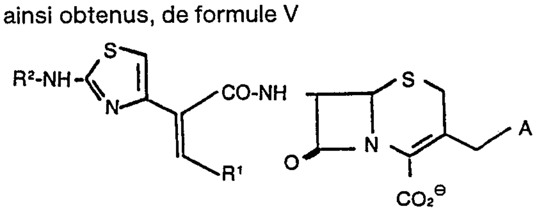

(V)

et éventuellement on transforme ces composés en les sels ou esters.

3. Procédé pour préparer des composés de formule générale I

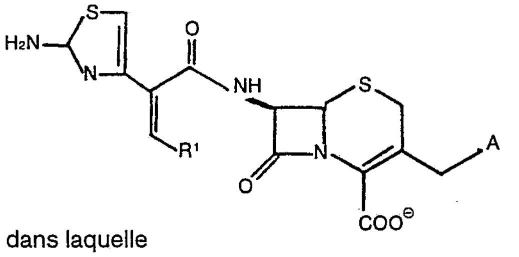

(I)

dans laquelle

$R^1$ et A ont le sens indiqué à la revendication 1, caractérisé en ce qu'on fait réagir des composés de formule IX

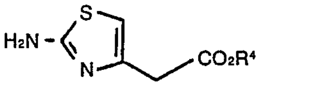

(IX)

dans laquelle

$R^4$ représente un reste alkyle, cycloalkyle, alcényle, cycloalcényle, aryle ou hétérocyclyle, éventuellement substitués avec un composé de formule

$$(R^5–O–CO)_2O$$

dans laquelle

$R^5$ a le sens indiqué ci-dessus pour $R^4$,

puis l'on fait réagir le composé ainsi obtenu, de formule X

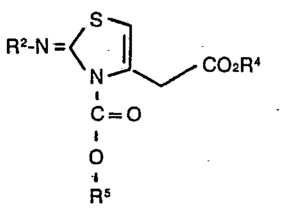

(X)

dans laquelle

$R^2$ représente le reste $R^5$–O–CO et $R^4$ et $R^5$ ont les sens indiqués ci-dessus,

tout d'abord avec un base puis avec un aldéhyde de formule

$$R^1–CHO$$

dans laquelle $R^1$ a le sens précité,

pour obtenir un composé de formule XI

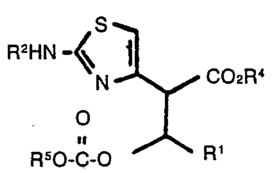

(XI)

dans laquelle $R^1$, $R^2$, $R^4$ et $R^5$ ont les sens précités, puis l'on fait réagir le composé XI avec une base pour obtenir le composé de formule XII

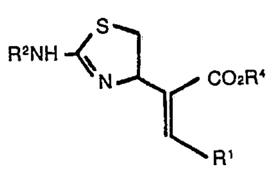

(XII)

dans laquelle

$R^1$, $R^2$, et $R^4$ ont les sens précités,

puis l'on saponifie pour obtenir un mélange des composés répondant aux formules II et IIa

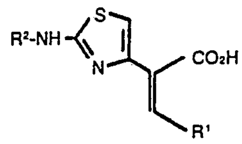

+

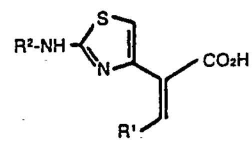

(II) (IIa)

ou l'on saponifie sélectivement pour obtenir les composés de formule II, $R^1$ et $R^2$ ayant à chaque fois les sens indiqués ci-dessus,

on transforme les composés de formule II en des composés de formule III

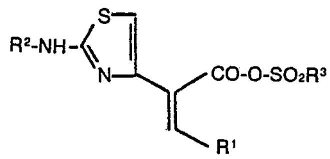

(III)

dans laquelle $R^3$ représente un reste alkyle comportant 1 à 10 atomes de carbone, qui peut éventuellement être substitué par du fluor, du chlore, un groupe CN, phényle, alkyloxycarbonyle, alkyloxy ou alkyle, ces derniers restes alkyle pouvant comporter 1 à 4 atomes de carbone, ou un reste phényle, qui peut éventuellement être substitué par du fluor, du chlore, du brome, un groupe CN, alkyle, alkyloxy, alkylthio, alkyloxycarbonyle (ces derniers groupes alkyle pouvant comporter 1 à 4 atomes de carbone), nitro, trifluorométhyle et phényle,

on transforme ensuite ces composés, selon la revendication 2, avec un composé de formule IV pour obtenir un composés de formule V et on enlève de celui-ci le groupe protecteur $R^2$ et éventuellement on le tranforme en les sels ou esters.

4. Procédé pour préparer des composés de formule générale I

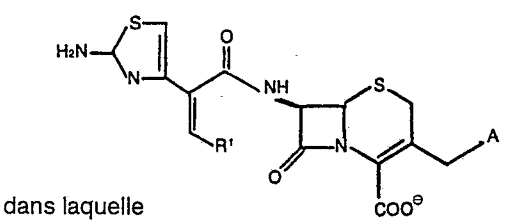

(I)

dans laquelle

$R^1$ et A ont le sens indiqué à la revendication 1, caractérisé en ce qu'on fait réagir des composés de formule XV

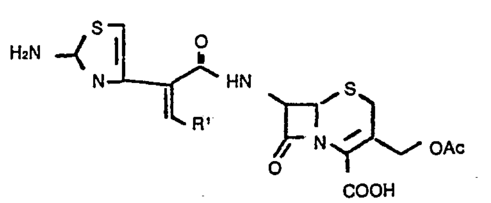

(XV)

avec un composés de formule A',

dans laquelle A' a le sens indiqué pour A à la revendication, mais ne porte pas la charge positive sur l'azote.

5. Médicament contenant au moins un composé selon l'une des revendications 1 à 4.

6. Composés selon la revendication 1, destinés à servir dans un procédé de traitement thérapeutique du corps humain ou animal.

7. Utilisation des composés selon la revendication 1, pour la préparation de médicaments.